(19) Europäisches Patentamt
European Patent Office
Office européen des brevets

(11) Publication number: **0 013 144**
**B1**

(12) # EUROPEAN PATENT SPECIFICATION

(45) Date of publication of patent specification: **23.02.83**

(21) Application number: **79302987.7**

(22) Date of filing: **20.12.79**

(51) Int. Cl.³: **C 07 C 93/14,**
C 07 C 143/74,
C 07 C 103/38,
C 07 C 103/78,
C 07 C 147/12,
C 07 C 161/00,
C 07 C 97/10,
C 07 C 143/80,
C 07 C 101/62,
C 07 C 153/09,
C 07 C 103/34

(54) **Substituted diphenylamine derivatives, compositions and herbicidal uses thereof, and processes for their preparation.**

(30) Priority: **22.12.78 AU 7207/78**
**27.03.79 AU 8191/79**
**08.06.79 AU 9113/79**

(43) Date of publication of application:
**09.07.80 Bulletin 80/14**

(45) Publication of the grant of the patent:
**23.02.83 Bulletin 83/8**

(84) Designated Contracting States:
**BE CH DE FR GB IT NL**

(56) References cited:
**EP - A - 0 000 156**
**FR - A - 2 348 182**
**GB - A - 916 242**
**GB - A - 2 022 578**

(73) Proprietor: **ICI AUSTRALIA LIMITED**
**ICI House 1 Nicholson Street P.O.Box 4311**
**Melbourne Victoria 3001 (AU)**

(72) Inventor: **Serban, Alexander**
**3 Maple Court**
**Doncaster, Victoria 3108 (AU)**
Inventor: **Watson, Keith Geoffrey**
**36 Medway Street**
**Box Hill North, Victoria 3129 (AU)**
Inventor: **Englin, Flora**
**24 Hillcrest Avenue**
**Chadstone, Victoria 3148 (AU)**

(74) Representative: **Fawcett, Richard Fennelly et al,**
**Imperial Chemical Industries PLC Legal**
**Department: Patents Thames House North**
**Millbank**
**London SW1P 4QG (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

EP 0 013 144 B1

Substituted diphenylamine derivatives, compositions and herbicidal uses thereof, and processes for their preparation

This invention relates to organic compounds having biological activity and in particular to organic compounds having herbicidal properties, to processes for the preparation of such compounds and to herbicidal compositions and processes utilizing such compounds.

We have now found a new class of diphenylamines which exhibit biological activity, and in particular herbidal activity.

Accordingly the invention provides a compound of formula II:

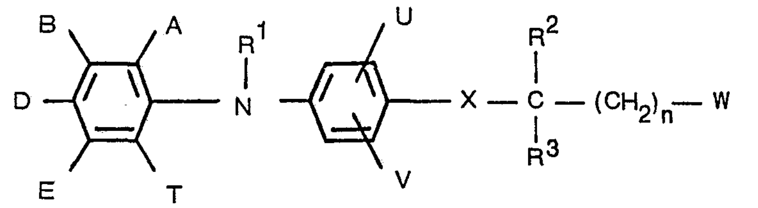

II

or a salt thereof wherein:

A, B, D, E and T are independently chosen from the group consisting of hydrogen, halogen, nitro, cyano, amino, formyl, $C_1$ to $C_6$ alkyl, $C_1$ to $C_6$ alkoxy, $C_1$ to $C_6$ haloalkyl, $C_1$ to $C_6$ haloalkoxy, ($C_1$ to $C_6$ alkoxy)carbonyl, $C_1$ to $C_6$ haloalkylthio, $C_1$ to $C_6$ haloalkylsulfinyl, $C_1$ to $C_6$ alkylsulfonyl, $C_1$ to $C_6$ haloalkylsulfonyl, sulfo, $C_1$ to $C_6$ alkoxysulfonyl, N,N-di($C_1$ to $C_6$ alkyl)carbamoyl, sulfamoyl and N,N-di($C_1$ to $C_6$ alkyl)sulfamoyl;

U and V are independently chosen from hydrogen, halogen, and nitro;

$R^1$ is chosen from the group consisting of hydrogen, $C_1$ to $C_6$ alkyl, $C_2$ to $C_6$ alkenyl, $C_2$ to $C_6$ alkynyl, $C_2$ to $C_6$ alkanoyl, benzyl, benzoyl and halobenzoyl;

$R^2$ and $R^3$ are independently chosen from hydrogen and $C_1$ to $C_6$ alkyl;

W is chosen from the groups

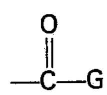 and $-CH_2Z$

wherein: G is chosen from the group consisting of hydroxy, mercapto, $C_1$ to $C_{10}$ alkoxy, $C_2$ to $C_{10}$ alkenyloxy, $C_2$ to $C_{10}$ alkynyloxy, $C_3$ to $C_7$ cycloalkoxy, $C_1$ to $C_{10}$ alkylthio, benzyloxy, N,N-di($C_1$ to $C_6$ alkyl)amino, $C_1$ to $C_{10}$ alkoxy substituted with a substituent chosen from halogen, amino, ammonio, N-($C_1$ to $C_6$ alkyl)amino, N,N-di($C_1$ to $C_6$ alkyl)amino, N,N,N-tri-($C_1$ to $C_6$ alkyl)ammonio, $C_1$ to $C_6$ alkoxy, $C_1$ to $C_6$ alkylthio, morpholino and 2-pyridyl, and the group OM wherein M is an alkali metal or alkaline earth metal ion; Z is chosen from the group consisting of hydroxy and $C_1$ to $C_6$ alkoxy;

X is oxygen; and

n is 0 or 2;

and provided that when:

B, E, T, U, V and $R^3$ are hydrogen;

D is trifluoromethyl or halogen;

$R^1$ is hydrogen, $C_1$ to $C_6$ alkyl, $C_2$ to $C_6$ alkenyl or benzyl;

$R^2$ is $C_1$ to $C_6$ alkyl;

W is the group

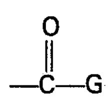

wherein G is chosen from hydroxy, mercapto, $C_1$ to $C_{10}$ alkoxy, $C_1$ to $C_{10}$ alkylthio or the group OM wherein M is an alkali metal ion:

X is oxygen; and

n is 0 or 2; then

A is not nitro.

The compounds of formula I wherein $R^2$ and $R^3$ are not the same, are optically active and the present invention also includes the individual stereo isomers of such compounds, and mixtures of those stereo isomers in addition to the racemic mixture of stereo isomers.

Preferred compounds of the invention fall into several sub-groups and include those in which:

(a) A is nitro;
D is chosen from hydrogen, nitro, cyano, $C_1$ to $C_6$ alkyl; $C_1$ to $C_6$ alkoxy and $C_1$ to $C_6$ haloalkoxy;
$R^1$ is chosen from hydrogen, $C_1$ to $C_6$ alkyl, $C_2$ to $C_6$ alkenyl, $C_2$ to $C_6$ alkynyl and benzyl;
$R^2$ is chosen from hydrogen and $C_1$ to $C_6$ alkyl;
B, E, T, U, V and $R^3$ are hydrogen;
W is chosen from the groups

$$-\overset{\displaystyle O}{\overset{\|}{C}}-G \quad \text{and} \quad -CH_2Z$$

wherein: G is chosen from hydroxy, mercapto, $C_1$ to $C_{10}$ alkoxy, $C_2$ to $C_{10}$ alkenyloxy, $C_2$ to $C_{10}$ alkynyloxy, $C_3$ to $C_7$ cycloalkoxy, $C_1$ to $C_{10}$ alkylthio, benzyloxy, N,N-di($C_1$ to $C_6$ alkyl)amino, the group OM wherein M is an alkali metal or alkaline earth metal ion, and the group $C_1$ to $C_{10}$ alkoxy substituted with a substituent chosen from $C_1$ to $C_6$ alkoxy, $C_1$ to $C_6$ alkylthio, halogen, N,N-di-($C_1$ to $C_6$ alkyl)amino, N,N,N-tri($C_1$ to $C_6$)ammonio and morpholino; and Z is chosen from hydroxy and $C_1$ to $C_6$ alkoxy;
X is oxygen; and
n is 0.

(b) A is chosen from hydrogen, halogen, $C_1$ to $C_6$ haloalkyl, cyano and amino;
D is chosen from hydrogen, nitro, cyano, halogen, $C_1$ to $C_6$ alkyl, $C_1$ to $C_6$ haloalkyl, $C_1$ to $C_6$ alkoxy and $C_1$ to $C_6$ haloalkoxy;
$R^1$ is chosen from hydrogen, $C_1$ to $C_6$ alkyl, $C_2$ to $C_6$ alkenyl, $C_2$ to $C_6$ alkynyl and benzyl;
$R^2$ is chosen from hydrogen and $C_1$ to $C_6$ alkyl;
B, E, T, U, V and $R^3$ are hydrogen;
W is chosen from the groups

$$-\overset{\displaystyle O}{\overset{\|}{C}}-G \quad \text{and} \quad -CH_2Z$$

wherein: G is chosen from hydroxy, mercapto, $C_1$ to $C_{10}$ alkoxy, $C_2$ to $C_{10}$ alkenyloxy, $C_2$ to $C_{10}$ alkynyloxy, cyclohexyloxy, $C_2$ to $C_{10}$ alkylthio, benzyloxy, N,N-di($C_1$ to $C_6$ alkyl)amino, the group OM wherein M is an alkali metal or alkaline earth metal ion, and the group $C_1$ to $C_{10}$ alkoxy substituted with a substituent chosen from $C_1$ to $C_6$ alkoxy, $C_1$ to $C_6$ alkylthio, halogen, N,N-di($C_1$ to $C_6$ alkyl)amino, N,N,N-tri($C_1$ to $C_6$ alkyl)ammonio, and morpholino; and Z is chosen from hydroxy and $C_1$ to $C_6$ alkoxy;
X is oxygen; and
n is 0.

(c) A is nitro;
D is chosen from $C_1$ to $C_6$ haloalkyl and halogen;
$R^1$ is chosen from hydrogen, $C_1$ to $C_6$ alkyl, $C_2$ to $C_6$ alkenyl and benzyl;
$R^2$ is chosen from hydrogen and $C_1$ to $C_6$ alkyl;
B, E, T, U, V and $R^3$ are hydrogen;
W is chosen from the groups

$$-\overset{\displaystyle O}{\overset{\|}{C}}-G \quad \text{and} \quad -CH_2Z$$

wherein: G is chosen from $C_2$ to $C_{10}$ alkenylxoy, $C_2$ to $C_{10}$ alkynyloxy, $C_3$ to $C_7$ cycloalkoxy, benzyloxy, N,N-di($C_1$ to $C_6$ alkyl)amino, $C_1$ to $C_{10}$ alkoxy substituted with a substituent chosen from $C_1$ to $C_6$ alkoxy, $C_1$ to $C_6$ alkylthio, halogen, N,N-di($C_1$ to $C_6$ alkyl)amino, N,N,N-tri($C_1$ to $C_6$ alkyl)ammonio and morpholino; and Z is chosen from hydroxy and $C_1$ to $C_6$ alkoxy;
X is oxygen; and
n is 0.

(d) A is nitro;
D is chosen from $C_1$ to $C_6$ haloalkyl and halogen;
$R^1$ is $C_2$ to $C_6$ alkynyl;
$R^2$ is chosen from hydrogen and $C_1$ to $C_6$ alkyl;
B, E, T, U, V and $R^3$ are hydrogen;
W is chosen from the groups

$$-\overset{O}{\overset{\|}{C}}-G \text{ and } -CH_2Z$$

wherein: G is chosen from hydroxy, mercapto, $C_1$ to $C_{10}$ alkoxy, $C_2$ to $C_{10}$ alkenyloxy $C_2$ to $C_{10}$ alkynyloxy, $C_3$ to $C_7$ cycloalkoxy, $C_1$ to $C_{10}$ alkylthio, benzyloxy, N,N-di($C_1$ to $C_6$ alkyl)amino, the group OM wherein M is an alkali metal or alkaline earth metal ion, and the group $C_1$ to $C_6$ alkoxy substituted with a substituent chosen from $C_1$ to $C_6$ alkoxy, $C_1$ to $C_6$ alkylthio, halogen, N,N-di($C_1$ to $C_6$ alkyl)amino, N,N,N-tri($C_1$ to $C_6$ alkyl)ammonio and morpholino; and Z is chosen from hydroxy and $C_1$ to $C_6$ alkoxy;

X is oxygen; and

n is 0.

More preferred compounds of the invention include those in which:

(e) A is nitro;

D is chosen from nitro, cyano, methyl, ethyl, methoxy and ethoxy;

$R^1$ is chosen from hydrogen, methyl, ethyl, n-propyl, n-butyl, allyl, propargyl and benzyl;

$R^2$ is methyl;

B, E, T, U, V and $R^3$ are hydrogen;

W is chosen from the groups

$$-\overset{O}{\overset{\|}{C}}-G \text{ and } -CH_2Z$$

wherein: G is chosen from hydroxy, $C_1$ to $C_6$ alkoxy, allyloxy, propargyloxy, $C_1$ to $C_4$ alkylthio, cyclohexyloxy, benzyloxy, N,N-dimethylamino, N,N-diethylamino, 2-haloethoxy, 2-($C_1$ to $C_6$ alkylthio)ethoxy, 2-(morpholino)ethoxy, 2-[N,N-di($C_1$ to $C_6$ alkyl)amino]ethoxy, 2-[N,N,N-tri($C_1$ to $C_6$ alkyl)ammonio]ethoxy and the groups OM wherein M is sodium or potassium; and Z is chosen from hydroxy and $C_1$ to $C_6$ alkoxy.

X is oxygen; and

n is 0.

(f) A is chosen from hydrogen, fluoro, chloro, bromo, trifluoromethyl and amino;

D is chosen from fluoro, chloro, bromo, nitro, and trifluoromethyl;

$R^1$ is chosen from hydrogen, methyl, ethyl, n-propyl, n-butyl, allyl, propargyl and benzyl;

$R^2$ is methyl;

B, E, T, U, V and $R^3$ are hydrogen;

W is chosen from the groups

$$-\overset{O}{\overset{\|}{C}}-G \text{ and } -CH_2Z$$

wherein: G is chosen from hydroxy, $C_1$ to $C_6$ alkoxy, allyloxy, propargyloxy, cyclohexyloxy, benzyloxy, N,N-dimethylamino, N,N-diethylamino, 2-haloethoxy, 2-($C_1$ to $C_6$ alkylthio)ethoxy, 2-(morpholino)-ethoxy, 2-[N,N-di($C_1$ to $C_6$ alkyl)amino]ethoxy, 2-[N,N,N-tri($C_1$ to $C_6$ alkyl)ammonio]ethoxy, and the group OM wherein M is sodium or potassium; and Z is chosen from hydroxy and $C_1$ to $C_6$ alkoxy.

X is oxygen; and

n is 0.

(g) A is nitro;

D is chosen from fluoro, chloro, bromo and trifluoromethyl;

$R^1$ is chosen from hydrogen, methyl, ethyl, n-propyl, n-butyl, allyl and benzyl;

$R^2$ is methyl;

B, E, T, U, V and $R^3$ are hydrogen;

W is chosen from the groups

$$-\overset{O}{\overset{\|}{C}}-G \text{ and } -CH_2Z$$

wherein: G is chosen from allyloxy, propargyloxy, cyclohexyloxy, benzyloxy, N,N-dimethylamino, N,N-diethylamino, 2-haloethoxy, 2-($C_1$ to $C_6$ alkylthio)ethoxy, 2-(morpholino)ethoxy, 2-[N,N-di($C_1$ to $C_6$ alkyl)amino]ethoxy and 2-[N,N,N-tri($C_1$ to $C_6$ alkyl)ammonio]ethoxy; and Z is chosen from hydroxy, fluoro, chloro, bromo and $C_1$ to $C_6$ alkoxy;

X is oxygen; and
n is 0.

(h) A is nitro;
D is chosen from fluoro, chloro, bromo and trifluoromethyl;
$R^1$ is propargyl;
$R^2$ is methyl;
B, E, T, U, V and $R^3$ are hydrogen;
W is chosen from the groups

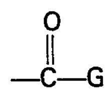 and —$CH_2Z$

wherein: G is chosen from hydroxy, $C_1$ to $C_6$ alkoxy, allyloxy, propargyloxy, cyclohexyloxy, benzyloxy, N,N-dimethylamino, N,N-diethylamino, 2-haloethoxy, 2-($C_1$ to $C_6$ alkylthio)ethoxy, 2-(morpholino)-ethoxy, 2-[N,N-di($C_1$ to $C_6$ alkyl)amino]ethoxy, 2-[N,N,N-tri($C_1$ to $C_6$ alkyl)ammonio]ethoxy and the group OM wherein M is sodium or potassium; and Z is chosen from hydroxy and $C_1$ to $C_6$ alkoxy.
X is oxygen; and
n is 0.

Examples of the compounds embraced by the invention include:

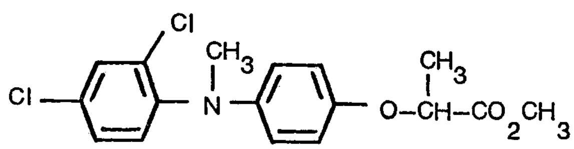 1

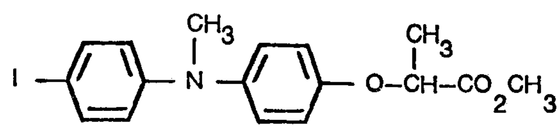 2

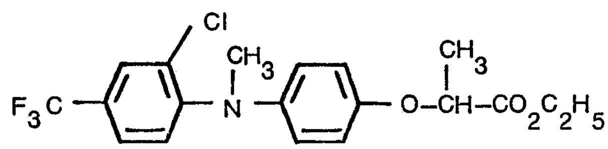 3

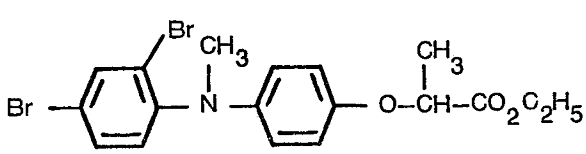 4

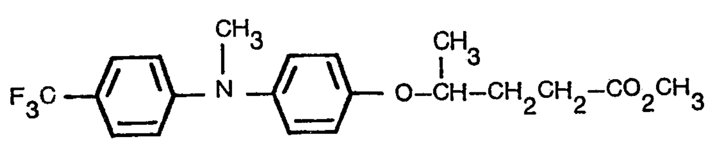 5

Specific examples of compounds of the invention include those compounds of formula III detailed in Table 1 below:

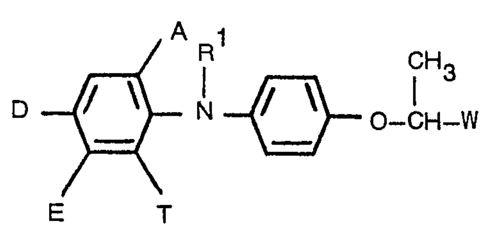 III

TABLE 1 PART A

| Compound Number | SUBSTITUENTS | | | | | |
|---|---|---|---|---|---|---|
| | A | D | E | T | $R^1$ | W |
| 1 | Cl | Cl | H | H | $CH_3$ | $CO_2CH_3$ |
| 6 | H | Cl | H | H | H | $CO_2CH_3$ |
| 7 | H | Cl | H | H | $CH_3$ | $CO_2CH_3$ |
| 8 | H | $CF_3$ | H | H | $CH_3$ | $CO_2CH_3$ |
| 9 | $NO_2$ | $NO_2$ | H | H | H | $CO_2CH_3$ |
| 10 | H | H | H | H | H | $CO_2CH_3$ |
| 11 | H | H | H | H | $CH_3$ | $CO_2CH_3$ |
| 12 | $NO_2$ | $NO_2$ | H | H | $CH_3$ | $CO_2CH_3$ |
| 13 | H | Cl | H | H | $COCH_3$ | $CO_2CH_3$ |
| 14 | H | Cl | H | H | $C_2H_5$ | $CO_2CH_3$ |
| 15 | Cl | Cl | H | H | H | $CO_2CH_3$ |
| 16 | $CF_3$ | $NO_2$ | H | H | H | $CO_2CH_3$ |
| 17 | $NO_2$ | H | H | H | H | $CO_2CH_3$ |
| 18 | $NO_2$ | $CH_3$ | H | H | H | $CO_2CH_3$ |
| 19 | H | $NO_2$ | H | H | H | $CO_2CH_3$ |
| 20 | $NO_2$ | $CF_3$ | H | $NO_2$ | H | $CO_2CH_3$ |
| 21 | $NO_2$ | Cl | Cl | H | H | $CO_2CH_3$ |
| 22 | $NO_2$ | $SO_2CH_3$ | H | H | H | $CO_2C_2H_5$ |
| 23 | $NO_2$ | $SO_2CH_3$ | H | H | $CH_3$ | $CO_2C_2H_5$ |
| 24 | $NO_2$ | Cl | Cl | H | $CH_3$ | $CO_2CH_3$ |
| 25 | $NO_2$ | $CF_3$ | H | Br | H | $CO_2CH_3$ |
| 26 | $NO_2$ | a | H | H | H | $CO_2CH_3$ |
| 27 | $NO_2$ | $CH_3O$ | H | H | H | $CO_2CH_3$ |
| 28 | $NO_2$ | $SO_2NH_2$ | H | H | H | $CO_2CH_3$ |
| 29 | $NO_2$ | CN | H | H | H | $CO_2CH_3$ |
| 30 | $NO_2$ | $SO_2NH_2$ | H | H | $CH_3$ | $CO_2CH_3$ |
| 31 | CN | $NO_2$ | H | H | H | $CO_2CH_3$ |
| 32 | $NO_2$ | $CF_3$ | H | H | H | $CO_2CH_2CH{=}CH_2$ |

TABLE 1 PART A (Continued)

| Compound No. | SUBSTITUENTS | | | | | |
|---|---|---|---|---|---|---|
| | A | D | E | T | $R^1$ | W |
| 33 | $NO_2$ | $CF_3$ | H | H | H | $CO_2$ 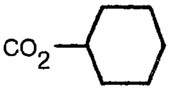 |
| 34 | $NO_2$ | $CF_3$ | H | H | H | $CO_2CH_2C_6H_5$ |
| 35 | $NO_2$ | H | H | H | $CH_3$ | $CO_2CH_3$ |
| 36 | $NO_2$ | b | H | H | H | $CO_2CH_3$ |
| 37 | $NO_2$ | $CO_2C_2H_5$ | H | H | H | $CO_2CH_3$ |
| 38 | $NO_2$ | $CF_3$ | H | H | H | $CO_2CH_2C{\equiv}CH$ |
| 39 | $NO_2$ | $CF_3$ | H | H | H | $CON(CH_3)_2$ |
| 40 | $NO_2$ | b | H | H | $CH_3$ | $CO_2CH_3$ |
| 41 | $NO_2$ | $CO_2C_2H_5$ | H | H | $CH_3$ | $CO_2CH_3$ |
| 42 | $NO_2$ | c | H | H | H | $CO_2CH_3$ |
| 43 | $NO_2$ | $SOCF_3$ | H | H | H | $CO_2CH_3$ |
| 44 | $SO_3H$ | $CF_3$ | H | H | H | $CO_2CH_3$ |
| 45 | $NO_2$ | $SO_2CF_3$ | H | H | H | $CO_2CH_3$ |
| 46 | $NO_2$ | $SO_2CF_3$ | H | H | H | $CO_2CH_3$ |
| 47 | $SO_2NH_2$ | $NO_2$ | H | H | H | $CO_2CH_3$ |
| 48 | $NO_2$ | CHO | H | H | H | $CO_2CH_3$ |
| 49 | b | $NO_2$ | H | H | H | $CO_2CH_3$ |
| 50 | $NO_2$ | $C_2H_5O$ | H | H | H | $CO_2CH_3$ |
| 51 | $NO_2$ | n-$C_3H_7O$ | H | H | H | $CO_2CH_3$ |
| 52 | $NO_2$ | $CF_3S$ | H | H | H | $CO_2C_2H_5$ |
| 53 | $SO_3CH_3$ | $CF_3$ | H | H | H | $CO_2CH_3$ |
| 54 | $NO_2$ | Cl | H | H | H | $CO_2$ 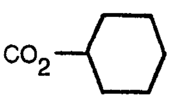 |
| 55 | $NO_2$ | $CF_3$ | H | H | $CH_3$ | $CO_2$ 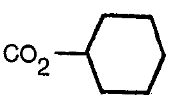 |
| 56 | $NO_2$ | $CF_3$ | H | H | $CH_2C{\equiv}CH$ | $CO_2CH_3$ |

TABLE 1 PART A (Continued)

| Compound Number | SUBSTITUENTS | | | | | |
|---|---|---|---|---|---|---|
| | A | D | E | T | $R^1$ | W |
| 57 | $NO_2$ | Cl | H | Cl | H | $CO_2C_2H_5$ |
| 58 | $NO_2$ | $CF_3$ | H | H | H | $CH_2OH$ |
| 59 | $NO_2$ | $CF_3$ | H | H | $CH_3$ | $CH_2OH$ |
| 60 | $NO_2$ | $CF_3$ | H | H | H | $CH_2OC_2H_5$ |
| 61 | $NO_2$ | $CF_3$ | H | H | $CH_3$ | $CH_2OC_2H_5$ |
| 62 | $NO_2$ | $CF_3$ | H | H | H | $CO_2CH_2CH_2N(CH_3)_2$ |
| 63 | $NO_2$ | Cl | H | H | H | $CO_2CH_2CH_2N(CH_3)_2$ |
| 64 | $NO_2$ | Cl | H | H | H | d |
| 65 | $NO_2$ | $CF_3$ | H | H | H | d |
| 66 | $NO_2$ | $CF_3$ | H | H | $COCH_3$ | $CO_2C_2H_5$ |
| 67 | $NO_2$ | CN | H | H | $CH_3$ | $CO_2C_2H_5$ |
| 68 | $NO_2$ | $CF_3$ | H | H | H | e |
| 69 | $NO_2$ | $CF_3$ | H | H | H | f |
| 70 | $NO_2$ | $CF_3$ | H | H | H | $CO_2CH_2CH_2Br$ |
| 71 | $CO_2CH_3$ | Cl | H | H | H | $CO_2C_2H_5$ |
| 72 | $NO_2$ | CN | H | H | H | $CO_2C_3H_7$-n |
| 73 | $NO_2$ | $CF_3$ | H | H | H | $CO_2CH_2CH_2SCH_3$ |
| 74 | $NO_2$ | $CF_3$ | H | H | g | $CO_2C_2H_5$ |
| 75 | $NO_2$ | $CF_3$ | H | Cl | H | $CO_2CH_3$ |
| 76 | $NO_2$ | $CF_3$ | H | H | H | h |

a. $HF_2C-ClFCO-$

b. $(CH_3)_2NSO_2-$

c. $(CH_3)_2NCO-$

d. $CO_2CH_2CH_2\overset{\oplus}{N}(CH_3)_3\overset{\ominus}{I}$

e. 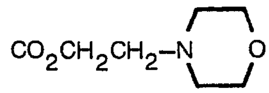

f. $CO_2CH_2CH_2N(C_2H_5)_2$

g. 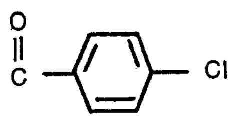

h. 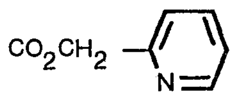

TABLE 1 PART B

| Compound Number | Structure |
|---|---|
| 77 | 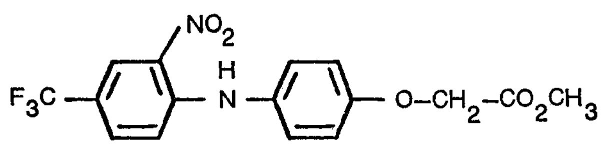 |
| 78 | 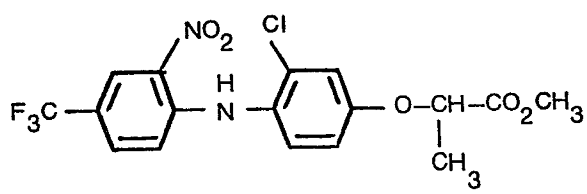 |
| 79 | 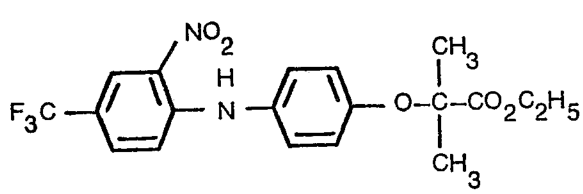 |

The compounds of the invention may be prepared by a variety of methods and in a further aspect the invention provides methods for the preparation of the compounds of formula II.

Compounds of formula IIa

$$(II;\ W=\overset{\overset{\large O}{\|}}{C}—G)$$

wherein G is not hydroxy may be prepared from the acid of formula IIb (II; W= $-CO_2H$) by any of the conventional methods known in the art for the conversion of a carboxylic acid to an acid salt, acid ester or acid amide. (SCHEME A)

SCHEME A

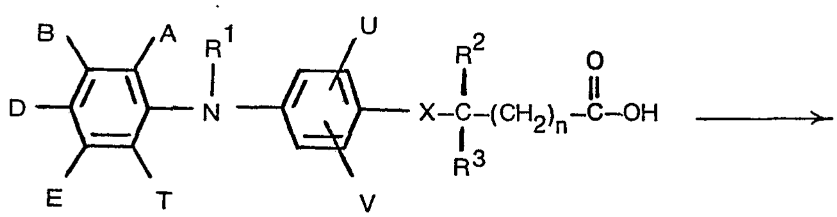

II b

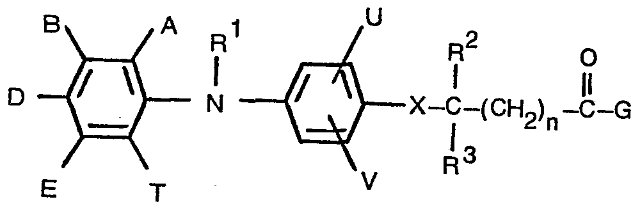

II a

Compounds of formula IIe (II; W= $-CH_2OH$) may be prepared from the acid or acid esters of formula If

$$(II;\ W = -\overset{O}{\overset{\|}{C}}-G \text{ wherein } G = OH \text{ or O-alkyl})$$

by any of the conventional methods known in the art for the conversion of an acid or acid ester to an alcohol (e.g. $LiAlH_4$ reduction) (SCHEME B).

SCHEME B

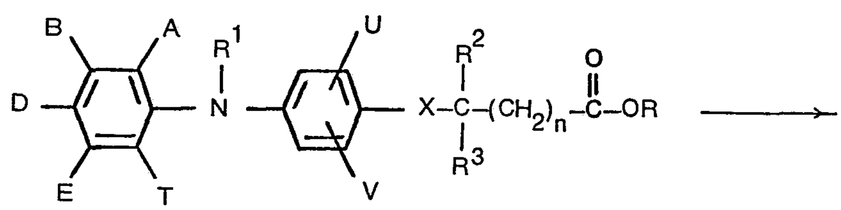

II f

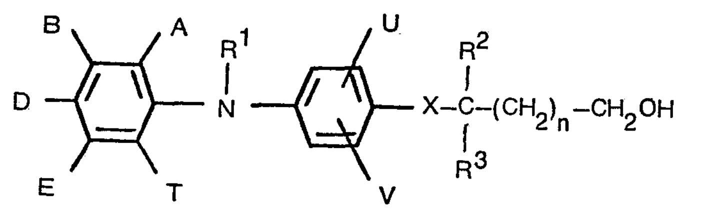

II e

Alcohols of formula IIe (II; W=—$CH_2OH$) may be converted to ethers (II; W=—$CH_2OR$) by any of the conventional methods known in the art.

Compounds of formula II wherein $R^1$ is not hydrogen may be prepared from compounds of formula II wherein $R^1$ is hydrogen by any of the conventional methods known in the art for the preparation of derivatives of secondary amines (e.g. alkylation and acylation).

Compounds of formula II wherein A, B, D, E, T, U, V, X, $R^1$, $R^2$, $R^3$, W and n are as hereinbefore defined may be prepared by the condensation of a phenol of formula IX with a compound of formula X wherein hal is chlorine, bromine or iodine, preferably in the presence of an alkaline material; according to SCHEME C.

SCHEME C

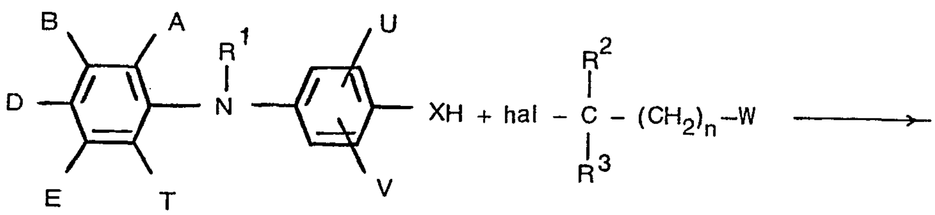

IX X

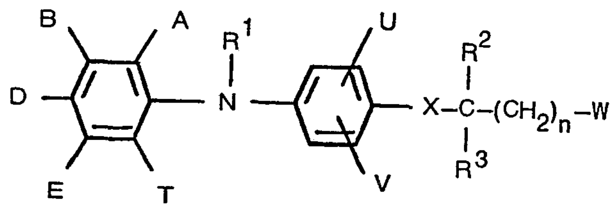

II

Compounds of formula II may also be prepared by:
a) the condensation of the appropriate benzene derivative of formula V, wherein L is a leaving group (for example, alkylsulfonyl, chlorine, bromine or iodine usually activated with respect to displacement) with the appropriate aniline of formula VI according to SCHEME D.

SCHEME D

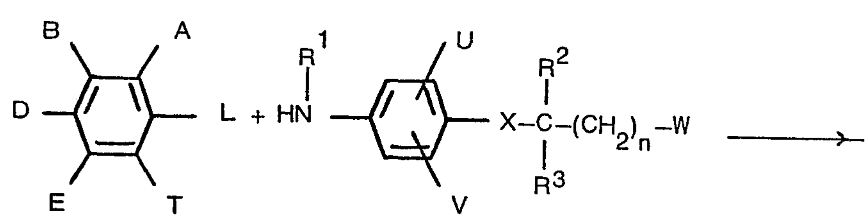

V VI

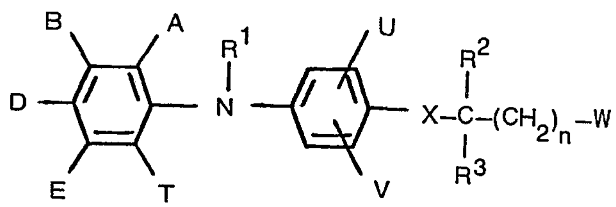

; or

II

b) the following steps in sequence:

(i) the condensation of the appropriate benzene derivative of formula V, wherein L is a leaving group (for example, alkylsulfonyl, chlorine, bromine or iodine usually activated with respect to displacement) with the appropriate aniline of formula VII, wherein Q is hydroxy or $C_1$ to $C_6$ alkoxy to give a compound of formula VIII wherein Q is hydroxy or $C_1$ to $C_6$ alkoxy;

(ii) the dealkylation of the compound of formula VIII prepared in step (i) above wherein Q is $C_1$ to $C_6$ alkoxy, to give a compound of formula IX; and

(iii) the condensation of the product of formula IX obtained in step (i) or step (ii) above with a compound of formula X according to the process described for SCHEME C above (Steps (i) and (ii) are shown in SCHEME E); or

c) the following steps in sequence:

(i) the condensation of the appropriate aniline derivative of formula XI with the appropriate benzene derivative of formula XII wherein L is a leaving group (for example, alkylsulfonyl, chlorine, bromine or iodine) and Q is hydroxy or $C_1$ to $C_6$ alkoxy to give a compound of formula VIII wherein Q is as hereinbefore defined;

(ii) the dealkylation of the compound of formula VIII prepared in step (i) above wherein Q is $C_1$ to $C_6$ alkoxy, to give a compound of formula IX according to the process described for SCHEME E step (ii) above; and

(iii) the condensation of the product of formula IX obtained in step (i) or step (ii) above with a compound of formula X according to the process described for SCHEME C above (step (i) is shown in SCHEME F).

SCHEME E

(i)

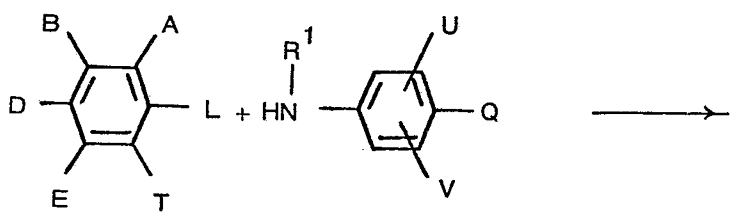

V VII

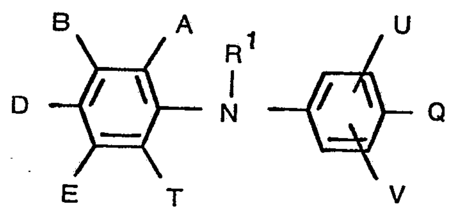

VIII

(ii)

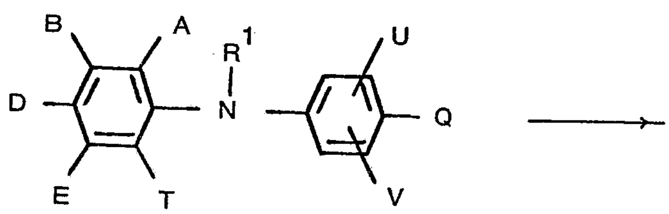

VIII

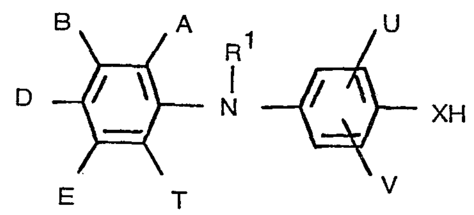

IX

SCHEME F

(i)

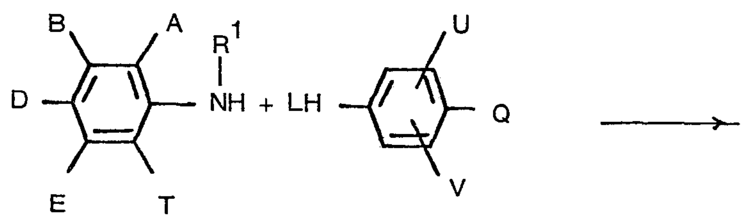

XI XII

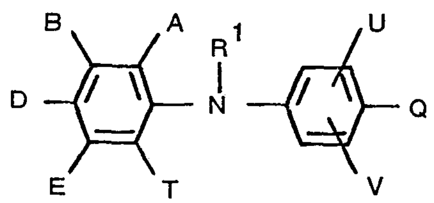

VIII

The condensation reaction illustrated in SCHEME C and outlined above is preferably carried out in the presence of an alkaline material and preferably in the presence of a solvent. Suitable alkaline materials include, for example, the alkali and alkaline earth metal hydroxides and carbonates such as sodium hydroxide, potassium hydroxide, sodium carbonate and potassium carbonate. Suitable solvents include ketones such as for example, acetone, methyl ethyl ketone and methyl isobutyl ketone, and dipolar aprotic solvents such as, for example, dimethylformamide, dimethylacetamide, dimethylsulfoxide, *N*-methylpyrrolidone, hexamethylphosphoramide and sulfolan.

The condensation reactions illustrated in SCHEMES D and E and outlined above are preferably carried out in the presence of a solvent.

The reaction conditions required to effect the condensation reactions illustrated in SCHEMES C, D, E and F and outlined above vary according to the nature of the reactants and the solvent used. In general the reaction is facilitated by the application of heat and usually a reaction temperature in the range of 40° to 150°C and reaction time of between 0.5 and 20 hours is satisfactory. However, higher or lower reaction temperatures and/or shorter or longer reaction times may be used if desired.

The dealkylation reactions illustrated in SCHEMES E and F and outlined in paragraphs b) (ii) and c) (ii) above may be effected using a variety of reagents known in the art. For example, aryl-alkyl ethers may be cleaved using reagents such as pyridine hdyrochloride, hydriodic acid, hydrobromic acid, sodium thioethoxide in dimethylformamide, acetyl *p*-toluene-sulphonate, sodium or potassium iodide in formic or acetic acid, lithium iodide in 2,4,6-collidine and boron tribromide. Reaction times and reaction conditions vary widely depending on the dealkylation agent used and the ether to be cleaved. The reaction conditions generally employed when using the above "ether-cleavage" reagents are known to those skilled in the art and may be adapted without undue experimentation to effect the "ether-cleavage" reactions illustrated in SCHEMES E and F and outlined in paragraph b) (ii) and c) (ii) above.

The compounds of the invention are active as herbicides and therefore, in a further aspect the invention provides a process for severely damaging or killing unwanted plants which process comprises applying to the plants, or to the growth medium of the plants, an effective amount of a compound of formula I as hereinbefore defined.

Generally speaking the compounds of the invention are herbicidally effective against a variety of plants. However, certain of the compounds of the invention are selectively active against monocotyledonous plants, dicotyledonous plants being relatively unaffected by rates of application of the compounds of the invention which are severely damaging or lethal to other plant species.

Therefore, in yet a further aspect the invention provides a process for selectively controlling the growth of weeds in crops which process comprises applying to the crop, or to the growth medium of the crop, a compound of formula I, as hereinbefore defined, in an amount sufficient to severely damage or kill plants but are preferably used in the form of a composition comprising a comound of the

The compounds of the invention may be applied directly to the plants (post-emergence application) or to the soil before the emergence of the plants (pre-emergence application). However, the compounds are, in general, more effective when applied to the plants post-emergence.

The compounds of formula I may be used on their own to inhibit the growth of, severely damage, or kill plants but are preferably used in the form of a composition comprising a compound of the invention in admixture with a carrier comprising a solid or liquid diluent. Therefore, in yet a further aspect the invention provides plant growth inhibiting, plant damaging, or plant killing compositions comprising a compound of formula I as hereinbefore defined and an inert carrier therefor.

Compositions according to the invention include both dilute compositions, which are ready for immediate use, and concentrated compositions, which require to be diluted before use, usually with water. Preferably the compositions contain from 0.01% to 90% by weight of the active ingredient. Dilute compositions ready for use preferably contain from 0.01 to 2% of active ingredient, while concentrated compositions may contain from 20 to 90% of active ingredient, although from 20 to 70% is usually preferred.

The solid compositions may be in the form of granules, or dusting powders wherein the active ingredient is mixed with a finely divided solid diluent, e.g. kaolin, bentonite, kieselguhr, dolomite, calcium carbonate, talc, powdered magnesia, Fuller's earth and gypsum. They may also be in the form of dispersible powders or grains, comprising a wetting agent to facilitate the dispersion of the powder or grains in liquid. Solid compositions in the form of a powder may be applied as foliar dusts.

Liquid compositions may comprise a solution or dispersion of an active ingredient in water optionally containing a surface-active agent, or may comprise a solution or dispersion of an active ingredient in a water-immiscible organic solvent which is dispersed as droplets in water.

Surface-active agents may be of the cationic, anionic, or non-ionic type. The cationic agents are, for example, quaternary ammonium compounds (e.g. cetyltrimethylammonium bromide). Suitable anionic agents are soaps; salts of aliphatic mono esters of sulphuric acid, for example sodium lauryl sulphate; and salts of sulphonated aromatic compounds, for example sodium dodecylbenzenesulphonate, sodium, calcium, and ammonium lignosulphonate, butylnaphthalene sulphonate, and a mixture of the sodium salts of diisopropyl- and triisopropylnaphthalenesulphonic acid. Suitable non-ionic agents are the condensation products of ethylene oxide with fatty alcohols such as oleyl alcohol and cetyl alcohol, or with alkylphenols such as octyl- or nonyl-phenol or octylcresol. Other non-ionic agents are the partial esters derived from long chain fatty acids and hexitol anhydrides, for example sorbitan monolaurate; the condensation products of the partial ester with ethylene oxide; and the lecithins.

The aqueous solutions or dispersions may be prepared by dissolving the active ingredient in water or an organic solvent optionally containing wetting or dispersing agent(s) and then, when organic solvents are used, adding the mixtures so obtained to water optionally containing wetting or dispersing agent(s). Suitable organic solvents include, for example, ethylene dichloride, isopropyl alcohol, propylene glycol, diacetone alcohol, toluene, kerosene, methylnaphthalene, the xylenes trichloroethylene.

The compositions for use in the form of aqueous solutions or dispersions are generally supplied in the form of a concentrate containing high proportion of the active ingredient, and the concentrate is then diluted with water before use. These concentrates are usually required to withstand storage for prolonged periods and after such storage, to be capable of dilution with water to form aqueous preparations which remain homogeneous for a sufficient time to enable them to be applied by conventional spray equipment. Concentrates conveniently contain 20—90%, preferably 20—70%, by weight of the active ingredient(s). Dilute preparations ready for use may contain varying amounts of the active ingredient(s) depending upon the intended purpose; amounts of 0.01% to 10% and preferably 0.1% to 2%, by weight of active ingredient(s) are normally used.

A preferred form of concentrated composition comprises the active ingredient which has been finely divided and which has been dispersed in water in the presence of a surface-active agent and a suspending agent. Suitable suspending agents are hydrophilic colloids and include, for example, polyvinylpyrrolidone and sodium carboxymethylcellulose, and the vegetable gums, for example gum acacia and gum tragacanth. Preferred suspending agents are those which impart thixotropic properties to, and increase the viscosity of the concentrate. Examples of preferred suspending agents include hydrated colloidal mineral silicates, such as montmorillonite, beidellite, nontronite, hectorite, saponite, and saucorite. Bentonite is especially preferred. Other suspending agents include cellulose derivatives and polyvinyl alcohol.

The rate of application of the compounds of the invention will depend on a number of factors including, for example, the compound chosen for use, the identity of the plants whose growth is to be inhibited the formulation selected for use and whether the compound is to be applied for foliage or root uptake. As a general guide, however, an application rate of from 0.005 to 20 kilograms per hectare is suitable while from 0.05 to 5 kilograms per hectare may be preferred.

The compositions of the invention may comprise, in addition to one or more compounds of the invention, one or more compounds not of the invention but which possess biological activity. For example, as hereinbefore indicated the compounds of the invention are in general substantially more effective against monocotyledonous plants or grass species than against dicotyledonous plants or broad-leaved species. As a result, in certain applications the herbicidal use of the compounds of the invention alone may be insufficient to protect a crop. Accordingly the herbicidal compositions can comprise a mixture of at least one herbicidal compound of formula I as hereinbefore defined with at least one other herbicide.

The other herbicide may be any herbicide not having the formula I. It will generally be a herbicide having a complementary action. For example, one preferred class is of mixtures comprising a herbicide active against broad-leaved weeds. A second preferred class is of mixtures comprising a contact herbicide.

Examples of useful complementary herbicides include:

A. benzo-2,1,3-thiadiazin-4-one-2,2-dioxides such as 3-isopropylbenzo-2,1,3-thiadiazin-4-one-2,2-dioxide (common name bentazon);
B. hormone herbicides and in particular the phenoxy-alkanoic acids such as 4-chloro-2-methylphenoxyacetic acid (common name MCPA), 2-(2,4-dichlorophenoxy)propionic acid (common name dichlorprop), 2,4,5-trichlorophenoxyacetic acid (common name 2,4,5-T), 4-(4-chloro-2-methylphenoxy)butyric acid (common name MCPB), 2,4-dichlorophenoxyacetic acid (common name 2,4-D), 4-(2,4-dichlorophenoxy)butyric acid (common name 2,4-DB), 2-(4-chloro-2-methylphenoxy)-propionic acid (common name mecoprop), and their derivatives (e.g. salts, esters, amides and the like);
C. 3-[4-(4-halophenoxy)phenyl]-1,1-dialkylureas such as 3-[4-(4-chlorophenoxy)phenyl]-1,1-dimethylurea (common name chloroxuron);
D. dinitrophenols and their derivatives (e.g. acetates) such as 2-methyl-4,6-dinitrophenol (common name DNOC), 2-tertiarybutyl-4,6-dinitrophenol (common name dinoterb), 2-secondarybutyl-4,6-dinitrophenol (common name dinoseb) and its ester dinoseb acetate;
E. dinitroaniline herbicides such as N',N'-diethyl-2,6-dinitro-4-trifluoromethyl-*m*-phenylenediamine (common name dinitramine), 2,6-dinitro-N,N-dipropyl-4-trifluoromethylaniline (common name trifluralin) and 4-methylsulfonyl-2,6-dinitro-N,N-dipropylaniline (common name nitralin);
F. phenylurea herbicides such as N'-(3,4-dichlorophenyl)-N,N-dimethylurea (common name diuron) and N,N-dimethyl-N'-[3-(trifluoromethyl)phenyl]urea (common name fluometuron);
G. phenylcarbamoyloxyphenylcarbamates such as 3-[(methoxycarbonyl)amino]phenyl (3-methylphenyl)carbamate (common name phenmedipham) and 3-[(ethoxycarbonyl)amino]phenyl phenylcarbamate (common name desmedipham);
H. 2-phenylpyridazin-3-ones such as 5-amino-4-chloro-2-phenylpyridazin-3-one (common name pyrazon);
I. uracil herbicides such as 3-cyclohexyl-5,6-trimethyleneuracil (common name lenacil), 5-bromo-3-*sec*-butyl-6-methyluracil (common name bromacil) and 3-*tert*-butyl-5-chloro-6-methyluracil (common name terbacil);
J. triazine herbicides such as 2-chloro-4-ethylamino-6-(*iso*-propylamino)-1,3,5-triazine (common name atrazine), 2-chloro-4,6-di(ethylamino)-1,3,5-triazine (common name simazine) and 2-azido-4-(*iso*-propylamino)-6-methylthio-1,3,5-triazine (common name aziprotryne);
K. 1-alkoxy-1-alkyl-3-phenylurea herbicides such as 3-(3,4-dichlorophenyl)-1-methoxy-1-methylurea (common name linuron), 3-(4-chlorophenyl)-1-methoxy-1-methylurea (common name monolinuron) and 3-(4-bromo-4-chlorophenyl)-1-methoxy-1-methylurea (common name chlorobromuron);
L. thiolcarbamate herbicides such as S-propyl dipropylthiocarbamate (common name vernolate);
M. 1,2,4-triazin-5-one herbicides such as 4-amino-4,5-dihydro-3-methyl-6-phenyl-1,2,4-triazine-5-one (common name metamitron) and 4-amino-6-*tert*-butyl-4,5-dihydro-3-methylthio-1,3,4-triazin-5-one (common name metribuzin);
N. benzoic acid herbicides such as 2,3,6-trichlorobenzoic acid (common name 2,3,6-TBA), 3,6-dichloro-2-methoxybenzoic acid (common name dicamba) and 3-amino-2,5-dichlorobenzoic acid (common name chloramben).
O. anilide herbicides such as N-butoxymethyl-$\alpha$-chloro-2',6'-diethylacetanilide (common name butachlor)- the corresponding N-methoxy compound (common name alachlor), the corresponding N-*iso*-propyl compound (common name propachlor) and 3',4'-dichloropropionanilide (common name propanil);
P. dihalobenzonitrile herbicides such as 2,6-dichlorobenzonitrile (common name dichlobenil), 3,5-dibromo-4-hydroxybenzonitrile (common name bromoxynil) and 3,5-diiodo-4-hydroxybenzonitrile (common name ioxynil).

Q. haloalkanoic herbicides such as 2,2-dichloropropionic acid (common name dalapon), trichloroacetic acid (common name TCA) and salts thereof;

R. diphenylether herbicides such as 4-nitrophenyl 2-nitro-4-trifluoromethylphenyl ether (common name fluorodifen), methyl 5-(2,4-dichlorophenoxy)-2-nitrobenzoate (common name bifenox), 2-nitro-5-(2-chloro-4-trifluoromethylphenoxy)benzoic acid and 2-chloro-4-trifluoromethylphenyl 3-ethoxy-4-nitrophenyl ether; and

S. miscellaneous herbicides including N,N-dimethyldiphenylacetamide (common name diphenamid), N-(1-naphthyl)phthalamic acid (common name naptalam) and 3-amino-1,2,4-triazole.

Examples of useful contact herbicides include:

T. bipyridylium herbicides such as those in which the active entity is the 1,1'-dimethyl-4,4'-dipyridylium ion (common name paraquat) and those in which the active entity is the 1,1'-ethylene-2,2'-dipyridylium ion (common name diquat);

U. organoarsenical herbicides such as monosodium methanearsonate (common name MSMA); and

V. amino acid herbicides such as N-(phosphonomethyl)glycine (common name glyphosate) and its salts and esters.

The invention is now illustrated by, but in no way limited to, the following Examples.

## Example 1

Methyl 2-[4-(4-chloroanilino)phenoxy]propionate (6)

a) Boron tribromide (2.5 ml) was added dropwise to a stirred solution of 4-chloro-4'-methoxydiphenylamine [3.0 g: prepared following E. A. Nodiff and M. Hausmann, J. Org. Chem., 29, 2453 (1964)] in dichloromethane (20 ml) held at a temperature of −78°C. After completion of the addition the solution was stirred at a temperature of −78°C for a period of 1 hr and was then allowed to warm to room temperature over a period of 2 hr. After stirring for a period of 4 hr at room temperature the solution was cooled and water (10 ml) was added cautiously to destroy the excess boron tribromide. Sodium hydrogen carbonate was added to the mixture until the evolution of carbon dioxide had ceased, the organic layer was separated and the aqueous layer was extracted with chloroform (2 × 50 ml). The combined organic layer and extracts were dried over anhydrous magnesium carbonate and the solvent was removed by distillation to give 4-(4-chloroanilino)phenol (2.8 g) as a pale grey solid.

b) A mixture of 4-(4-chloroanilino)phenol (2.8 g), methyl 2-bromopropionate (2.4 g), anhydrous potassium carbonate (2.0 g) and methyl ethyl ketone (50 ml) was heated under reflux with stirring for a period of 5 hr. The reaction mixture was poured into water (200 ml) and the aqueous mixture was extracted with chloroform (2 × 100 ml). The combined extracts were dried over anhydrous magnesium sulphate and the solvent was removed by distillation to give methyl 2-[4-(4-chloroanilino)phenoxy]-propionate (2.5 g) as a pale grey solid m.p. 103°C.

## Example 2

Methyl 2[4-(4-chloro-N-methylanilino)phenoxy]propionate (7)

a) A solution of 4-chloro-4'-methoxydiphenylamine (3.0 g) in toluene (30 ml) was treated with sodium hydride (1.0 g) and the mixture was heated under reflux for a period of 18 hr. Methyl iodide (3 ml) was added and the reaction mixture was heated under gentle reflux for a further period of 5 hr. The cooled solution was washed with water and the organic phase was separated and dried over anhydrous magnesium sulfate. The solvent was removed by distillation under reduced pressured to give a brown oil (2.6 g) which was purified by column chromatography over silica gel (eluant n-hexane/chloroform 4:1). The product 4-chloro-4'-methoxy-N-methyldiphenylamine (1.4 g), was obtained as a pale cream solid m.p. 69°C.

b) 4-(4-Chloro-N-methylanilino)phenol was prepared from 4-chloro-4'-methoxy-N-methyldiphenylamine following essentially the same procedure as that described in Example 1 part a).

c) Methyl 2-[4-(4-chloro-*N*-methylanilino)phenoxy]propionate was prepared from 4-(4-chloro-N-methylanilino) phenol and methyl 2-bromopropionate following essentially the same procedure as that described in Example 1 part b). The product, a colourless oil, was characterised by its p.m.r. spectrum and the details are recorded in Example 20, Table 2.

## Example 3

Methyl 2-[4-(4-trifluoromethyl-N-methylanilino)phenoxy]propionate (8)

a) A mixture of 4-bromobenzotrifluoride (18 g), p-anisidine (5 g), potassium carbonate (5.4 g) and cuprous iodide (1.2 g) was heated under reflux for a period of 24 hr. After cooling the mixture was diluted with chloroform (200 ml) and filtered through Celite ("Celite" is a Trade Mark). Excess 4-bromobenzotrifluoride was removed by distillation under reduced pressure and the residue was chromatographed over silica gel (eluant dichloromethane). The product, 4-trifluoromethyl-4'-methoxydiphenylamine, was obtained as a red oil which crystallised on standing to give a low melting point solid (2.0 g).

b) 4-Trifluoromethyl-4'-methoxy-N-methyldiphenylamine was prepared from 4-trifluoromethyl-4′-methoxydiphenylamine and methyl iodide following essentially the same procedure as that described in Example 2 part a).

c) 4-(4-Trifluoromethyl-N-methylanilino)phenol was prepared following essentially the same procedure as that described in Example 1 part a).

d) Methyl 2-[4-(4-trifluoromethyl-N-methylanilino)phenoxy]propionate was prepared from 4-(N-methyl-4-trifluoromethylanilino)phenol and methyl 2-bromopropionate following essentially the same procedure as that described in Example 1 part b). The product, a pale orange oil, was characterised by its p.m.r. spectrum and the details are recorded in Example 20, Table 2.

Example 4

The compounds no 9, 10, 13, 14 and 15 were prepared following essentially the same procedure as that described in Example 1 for the preparation of compound no 6. The compound no 31 was prepared following essentially the same procedure as that described in Example 3 parts a), c) and d).

The compounds were characterised by p.m.r. spectroscopy and the details are recorded in Example 20, Table 2.

Example 5

Methyl 2-[4-methylanilino)phenoxy]propionate (11)

Sodium hydride (0.5 g) was slowly added to a solution of methyl 2-[4-anilinophenoxy]propionate (3.5 g) in dry toluene (30 ml). The mixture was stirred for period of 1 hr and then methyl iodide (3 ml) was added. The mixture was heated to a temperature of 60°C under an inert atmosphere and stirred overnight at that temperature. After cooling the mixture was carefully treated with water and then washed with water. The organic phase was separated, dried over anhydrous magnesium sulfate and the solvent was removed by distillation under reduced pressure. The crude product was purified by chromatography over silica gel (eluant chloroform) to give a brown oil (1.9 g) which was characterised by its p.m.r. spectrum and the details are recorded in Example 20, Table 2.

Example 6

The compounds nos. 12, 23, 24, 33, 40, 41, 46, 56, 66, 67 and 74 were prepared following essentially the same procedure as that described in Example 5 for the preparation of compound no. 11. The compounds were characterised by p.m.r. spectroscopy and details of the spectra of the compounds or, in the case of solids, the melting points of the compounds are recorded in Example 20, Table 2.

Example 7

Methyl 2-[4-(2,4-dichloro-N-methylanilino)phenoxy]propionate (1)

a) A mixture of 2,4-dichloro-N-methylaniline (12.0 g), 4-bromoanisole (40.0 g), copper bronze (0.1 g), cuprous bromide (0.1 g) and anhydrous potassium carbonate (9.42 g) was heated at a temperature of 200°C for a period of 30 hr. After cooling the unreacted 4-bromoanisole was distilled from the reaction mixture under reduced pressure and dichloromethane was added to the residue. The mixture was washed with dilute aqueous hydrochloric acid and then with water and the organic phase was separated and dried ovr anhydrous magnesium sulfate. The solvent was removed by distillation and the crude product was purified by chromatography over silica gel (eluant chloroform) to give 2,4-dichloro-4′-methoxy-N-methyldiphenylamine (3.6 g).

b) 4-(2,4-Dichloro-N-methylanilino)phenol was prepared from 2,4-dichloro-4′-methoxy-N-methyldiphenylamine following essentially the same procedure as that described in Example 1 part a).

c) Methyl 2-[4-(2,4-dichloro-N-methylanilino)phenoxy]propionate was prepared from 4-(2,4-dichloro-N-methylanilino) phenol and methyl 2-bromopropionate following essentially the same procedure as that described in Example 1 part b). The product, a pale brown oil, was characterised by its p.m.r. spectrum and the details are recorded in Example 20, Table 2.

Example 8

Methyl 2-[4-(2-nitro-4-trifluoromethylanilino)phenoxy]propionate (21)

a) 4-(2-Nitro-4-trifluoromethylanilino)phenol was prepared from 4-chloro-3-nitrobenzotrifluoride and 4-aminophenol following the procedure described by Day and Peters in *J. Soc. Dyers Colour., 83,* 137 (1967).

b) Methyl 2-[4-(2-nitro-4-trifluoromethylanilino)phenoxy]propionate was prepared from 4-(2-nitro-4-trifluoromethylanilino)phenol and methyl bromopropionate following essentially the same procedure as that described in Example 1 part b) to give, as a red oil, methyl 2-[4-(2-nitro-4-trifluoromethylanilino)phenoxy]propionate.

The compounds no. 16, 17, 18, 19, 20, 21, 22, 25, 26, 27, 28, 29, 36, 37, 42, 43, 44, 45, 47, 48, 49, 50, 51, 52, 57, 75, 77 and 80 were prepared following essentially the same procedure as that described in part b) above. The compounds were characterised by p.m.r. spectroscopy and details of the spectra of the compounds or, in the case of solids, the melting points of the compounds are recorded in Example 20, Table 2.

Example 9

A mixture of thionyl chloride (15 ml) and 2-[4-(2-nitro-4-trifluoromethylanilino)phenoxy]-propionic acid (2.0 g) was boiled under reflux for a period of 2 hours. The excess thionyl chloride was removed by distillation and n-butanol (2 ml) was added to the residue with stirring. After stirring for a period of 1 hour at a temperature of 20°C the excess butanol was removed by distillation under reduced pressure. The residue was chromatographed over silica gel with dichloromethane elution to give n-butyl 2-[4-(2-nitro-4-trifluoromethylanilino)phenoxy]propionate as an orange solid (2.04 g), mp 67°C.

The compounds no. 32, 33, 34, 38, 39, 54, 55, 62, 63, 68, 69 70, 73 and 76 were prepared following essentially the same procedure as that described above. The compounds were characterised by p.m.r. spectroscopy and the details are recorded in Example 20, Table 2.

Example 10

2-(N,N,N-Trimethylammonio)ethyl 2-[4-(2-nitro-4-chloroanilino)phenoxy]propionate iodide salt (64)

A mixture of 2-(N,N-dimethylamino)ethyl 2-[4-(2-nitro-4-chloroanilino)phenoxy]propionate (1.0 g, Example 9), methyl iodide (1 ml) and dichloromethane (10 ml) was stirred at a temperature of 20°C for a period of 2 hours. The solvent was removed by distillation under reduced pressure to give the title compound as an orange solid (1.3 g), mp 138—140°C.

Example 11

2-(N,N,N-Trimethylammonio)ethyl 2-[4-(2-nitro-4-trifluoromethylanilino)phenoxy]propionate iodide salt (65) was prepared from, 2-(N,N-dimethylamino)ethyl 2-[4-(2-nitro-4-trifluoromethylanilino)-phenoxy]propionate following essentially the same procedure as that described in Example 10 for the preparation of compound no 64. The product had a melting point of 156°C.

Example 12

Methyl 2-[4-(N-methyl-2-nitro-4-sulfonamidoanilino)phenoxy]propionate (30)

a) A mixture of 4-chloro-3-nitrobenzenesulfonamide (2.3 g), sodium acetate (1.6 g), 4-(N-methylamino)phenol sulfate (1.7 g) and water (50 ml) was boiled under reflux for a period of 20 hours. After cooling, the mixture was extracted with ethyl acetate (2 × 150 ml) and the organic layer was separated and extracted with 1M sodium hydroxide solution. The aqueous layer was separated, acidified and extracted with ethyl acetate (2 × 200 ml). The extracts were dried (anhydrous $MgSO_4$) and the solvent was evaporated to give 4-(2-nitro-4-sulfonamido-N-methylanilino)phenol (2.9 g).

b) Methyl 2-[4-(2-nitro-4-sulfonamide-N-methylanilino)phenoxy]propionate was prepared from 4-(2-nitro-4-sulfonamido-N-methylanilino)phenol and methyl 2-bromopropionate essentially as described in Example 1 part b). The product, an oil, was characterized by its p.m.r. spectrum and the details are recorded in Example 20, Table 2.

Example 13

Methyl 2-[2-chloro-4-(2-nitro-4-trifluoromethylanilino)phenoxy]propionate (73)

A mixture of methyl 2-[4-(2-nitro-4-trifluoromethylanilino)phenoxy]propionate (5.0 g, Example 8), N-chlorosuccinimide (1.9 g) and chlorobenzene (50 ml) was heated under reflux for a period of 24 hours. The chlorobenzene solvent was removed by distillation under reduced pressure and the residue was dissolved in dichloromethane and the solution was washed with water. The dichloromethane solution was dried (anhydrous $MgSO_4$) and the solvent was removed by evaporation under reduced pressure. The residue was chromatographed over silica gel with dichloromethane elution to give the title compound (3.5 g) as a red oil. The compound was characterized by its p.m.r. spectrum and the details are recorded in Example 20, Table 2.

Example 14

2-[4-(2-Nitro-4-trifluoromethylanilino)phenoxy]propanol (58)

A solution of methyl 2-[4-(2-nitro-4-trifluoromethylanilino)phenoxy]propionate (25 g Example 8) in anhydrous diethyl ether (100 ml) was added dropwise to a stirred mixture of powdered lithium aluminium hydride (2.0 g) and anhydrous diethyl ether (40 ml). After completion of the addition the mixture was heated under reflux for a period of 1 hour. Excess lithium aluminium hydride was destroyed by successively treating the mixture with ethyl acetate (3 ml) and aqueous 5M hydrochloric acid (3 ml). The mixture was continuously extracted with diethyl ether to separate the organic material. The solvent was removed by distillation and the residue was chromatographed over silica gel to give the title compound (15 g) as a red oil. The compound was characterized by its p.m.r. spectrum and the details are recorded in Example 20, Table 2.

Example 15

2-[4-(N-methyl-2-nitro-4-trifluoromethylanilino)phenoxy]propanol (59) was prepared from methyl 2-[4-(N-methyl-2-nitro-4-trifluoromethylanilino)phenoxy]propionate (Example 6) following essentially the

same procedure as that described in Example 14. The product, an oil, was characterized by its p.m.r. spectrum and the details are recorded in Example 20, Table 2.

Example 16

Ethyl 2-[4-(2-nitro-4-trifluoromethylanilino)phenoxy]propyl ether (60)

A mixture of 2-[4-(2-nitro-4-trifluoromethylanilino)phenoxy]propanol (1.0 g, Example 14), ethyl iodide (1.0 g), potassium carbonate (0.5 g) and dimethylformamide (20 ml) was heated at a temperature of 67°C for a period of 24 hours. The mixture was poured into water (30 ml) and the aqueous mixture was extracted with diethyl ether. The ether extracts were dried (anhydrous $MgSO_4$) and the solvent was evaporated under reduced pressure. The residue was chromatographed over silica gel to give the title compound (0.7 g) as a red oil. The compound was characterized by its p.m.r. spectrum and the details are recorded in Example 20, Table 2.

Example 17

Ethyl 2-[4-(N-methyl-2-nitro-4-trifluoromethylanilino)phenoxy]propyl ether (61) was prepared from 2-[4-N-methyl-2-nitro-4-trifluoromethylanilino)phenoxy]propanol (Example 15) following essentially the same procedure as that described in Example 16.

Example 18

Ethyl 2-[4-(4-chloro-3-methoxycarbonylanilino)phenoxy]propionate (71)

a) 5-Chloro-N-(4-methoxyphenyl)anthranilic acid was prepared according to the method of Ledochowski et al (*Chem. Abstract, 59,* 570 (1963)).

A mixture of 5-chloro-N-(4-methoxyphenyl)anthranilic acid (2 g), anhydrous potassium carbonate (1 g) and dimethyl sulphate (1 ml) in acetone (20 ml) was boiled under reflux for 3 hr. The acetone was removed under reduced pressure and the residue partitioned between chloroform and water. The chloroform layer was dried and concentrated to give methyl 5-chloro-N-(4-methoxyphenyl) anthranilate (1.8 g) as a pale yellow crystalline solid, mp 87°C.

b) Methyl 5-chloro-N-(4-hydroxyphenyl)anthranilate was prepared from methyl 5-chloro-N-(4-methoxyphenyl)anthranilate following essentially the same procedure as that described in Example 1 part a).

c) Ethyl 2-[4-(4-chloro-2-methoxycarbonylphenylamino)phenoxy]propionate was prepared from methyl 5-chloro-N-(4-hydroxyphenyl)anthranilate and ethyl 2-bromopropionate following essentially the same procedure as that described in Example 1 part b). The product, a pale yellow oil, was characterized by its p.m.r. spectrum and the details are recorded in Example 20, Table 2.

Example 19

Propyl 2-[4-(4-cyano-2-nitroanilino)phenoxypropionate (72)

A solution of methyl 2-[4-(4-cyano-2-nitroanilino)phenoxy]propionate (1.2 g) and p-toluenesulphonic acid (40 mg) in propanol (90 ml) was boiled under reflux for 48 hr. The propanol was removed under reduced pressure and the residue partitioned between chloroform and dilute aqueous sodium bicarbonate. The chloroform layer was dried and evaporated to give propyl 2-[4-(4-cyano-2-nitroanilino)phenoxy]propionate as a red oil which was characterized by its p.m.r. spectrum and the details are recorded in Example 20, Table 2.

Example 20

The majority of the compounds of the invention are oils and were characterised by and may be identified by p.m.r. spectroscopy. For convenience the p.m.r. spectroscopy data, mass spectrometric data, melting points and boiling points, where appropriate are recorded in Table 2 below.

TABLE 2 PART A

| Compound No. | PROTON CHEMICAL SHIFT δ IN PPM ($CDCl_3$) | | | | | |
|---|---|---|---|---|---|---|
| | Phenyl | A, D Groups | $R^1$ | $R^2$ | $R^3$ | W Group |
| 1 | 7.0,m,7H | | 3.2,s, 3H | 1.6,d, 3H | 4.8,q, 3H | 3.8,s, 3H |
| 7 | 6.6–7.4,m,8H | | 3.2,s, 3H | 1.6,d, 3H | 4.8,q, 1H | 3.7,s, 3H |
| 8 | 6.6–7.6,m,8H | | 3.3,s, 3H | 1.6,d, 3H | 4.8,q, 1H | 3.8,s, 3H |
| 9 | 7.0,m,4H 8.1–9.1,m,3H | | 9.8,s, 1H | 1.7,d, 3H | 4.9,q, 1H | 3.8,s, 3H |
| 10 | 6.9,m,9H | | 5.5,s, 1H | 1.5,d, 3H | 4.6,q, 1H | 3.5,s, 3H |
| 11 | 7.1,m,9H | | 3.3,s, 3H | 1.7d, 3H | 4.9,q, 1H | 3.8,s, 3H |
| 12 | 7.1,m,4H 8.5,m,3H | | 3.5,s, 3H | 1.7,d, 3H | 4.9,q, 1H | 3.8,s, 3H |
| 13 | 6.9–7.5,m,8H | | 2.0,s, 3H | 1.6,d, 3H | 4.8,q, 1H | 3.8,s, 3H |
| 14 | 6.6–7.3,m,8H | | 3.7,q, 2H 1.1,t, 3H | 1.6,d, 3H | 4.8,q, 1H | 3.8,s, 3H |
| 15 | 6.6–7.3,m,8H | | 6.0,s, 1H | 1.6,d, 3H | 4.8,q, 1H | 3.8,s, 3H |
| 16 | 6.7–7.3,m,5H 8.3,d,1H 8.6,br.s,1H | | 7.0,s, 1H | 1.7,d, 3H | 4.8,q, 1H | 3.8,s,3H |
| 17 | 6.6–7.5,m,7H 8.3,d,1H | | 9.5,s, 1H | 1.6,d, 3H | 4.8,q, 1H | 3.8,s,3H |
| 18 | 6.7–7.4,m,6H 8.1,br.s,1H | 2.3,s, 3H | 9.4,s, 1H | 1.6,d, 3H | 4.8,q, 1H | 3.8,s,3H |
| 21 | 6.8–7.3,m,5H 8.2,s,1H | | 9.3,s, 1H | 1.6,d, 3H | 4.8,q, 1H | 3.8,s,3H |
| 35 | 6.7–7.9,m,8H | | 3.3,s, 3H | 1.6,d, 3H | 4.8,q, 1H | 3.8,s,3H |
| 22 | 7.0–7.4,m,5H 7.9,d,1H 8.8,d,1H | 3.1,s, 3H | 9.9,s, 1H | 1.7,d, 3H | 4.9,q, 1H | 1.3,t,3H 4.3,q,2H |
| 23 | 7.0,dofd,4H 7.3–8.3,m,3H | 3.1,s, 3H | 3.3,s, 3H | 1.5,d, 3H | 4.8,q, 1H | 1.15,t,3H 4.2,q,2H |

TABLE 2 PART A (Continued)

| Compound No. | PROTON CHEMICAL SHIFT δ IN PPM ($CDCl_3$) | | | | | |
|---|---|---|---|---|---|---|
| | Phenyl | A, D Groups | $R^1$ | $R^2$ | $R^3$ | W Group |
| 24 | 6.9,s,4H<br>7.4,s,1H<br>7.9,s,1H | | 3.3,s,<br>3H | 1.6,d,<br>3H | 4.8,q,<br>1H | 3.8,s,3H |
| 25 | 7.1,s,4H<br>8.3,s,1H<br>8.6,d,1H | | 8.7,s,<br>1H | 1.6,d,<br>3H | 4.9,q,<br>1H | 3.9,s,3H |
| 26 | 6.6–7.5,m,6H<br>8.2,s,1H | 6.6–<br>7.5,m,<br>2H | 9.6,s,<br>1H | 1.7,d,<br>3H | 4.9,q,<br>1H | 3.9,s,3H |
| 27 | 6.8–7.6,m,7H | 7.9,s,<br>1H | 9.5,s,<br>1H | 1.6,d,<br>3H | 4.8,q,<br>1H | 3.8,s,3H |
| 30 | 7.0,dofd, 4H<br>7.1–8.4,m,3H | 5.6,s,<br>2H | 3.3,s,<br>3H | 1.6,d,<br>3H | 4.8,q,<br>1H | 3.8,s,3H<br>3H |
| 32 | 6.9–7.7,m,6H<br>8,5,s,1H | | 9.75,s,<br>1H | 1.7,d,<br>3H | 4.9–<br>5.5,m,<br>1H | 4.7,d,2H<br>4.9–5.5,<br>m,2H<br>5.7–6.2,<br>m,1H |
| 33 | 6.6–7.7,m,6H<br>8.5,s,1H | | 9.7,s, | 1.1–<br>2.3,m,<br>3H | 4.8,q,<br>1H | 1.1–2.3,<br>m,10H |
| 34 | 6.8–7.8,m,6H<br>8.5,s,1H | | 9.7,s,<br>1H | 1.7,d,<br>3H | 4.9,q,<br>1H | 5.3,s,2H<br>6.8–7.8,<br>m,5H |
| 36 | 6.8–7.3,m,5H<br>7.6,dofd,1H<br>8.6,d,1H | 2.8,s,<br>6H | 9.7,s,<br>1H | 1.6,d,<br>3H | 4.8,q,<br>1H | 3.7,s,3H |
| 38 | 6.9–7.7,m,6H<br>8,5,d,1H | | 9.7,s,<br>1H | 1.7,d,<br>3H | 4.8,q,<br>1H | 2.5,t,1H<br>4.8,d,2H |
| 39 | 6.9–7.8,m,6H<br>8.6,s,1H | | 9.7,s,<br>1H | 1.7,d,<br>3H | 5.0,q,<br>1H | 3.2,s,6H |
| 41 | 6.8–7.5,m,4H<br>8.0–8.5,m,3H | 1.4,t,<br>3H<br>4.2,q,<br>2H | 3.4,s,<br>3H | 1.7,d,<br>3H | 4.8,q,<br>1H | 3.8,s,3H |
| 43 | 6.8–7.7,m,6H<br>8.5,s,1H | | 9.7,s,<br>1H | 1.5,d,<br>3H | 4.7,q,<br>1H | 3.7,s,3H |
| 45 | 6.9–7.9,m,6H<br>8.8,s,1H | | 10.1,s,<br>1H | 1.6,d,<br>3H | 4.9,q,<br>1H | 3.8,s,3H |
| 46 | 6.7–8.3,m,7H | | 3.5,s,<br>3H | 1.5,d,<br>3H | 4.9,q,<br>1H | 3.8,s,3H |

TABLE 2 PART A (Continued)

| Compound No. | PROTON CHEMICAL SHIFT δ IN PPM ($CDCl_3$) Phenyl | A, D Groups | $R^1$ | $R^2$ | $R^3$ | W Group |
|---|---|---|---|---|---|---|
| 47 | 6.8–7.3,m,5H<br>8.1,dofd,1H<br>8.6,d,1H | 5.9,s,<br>2H | 8.3,s,<br>1H | 1.6,d,<br>3H | 4.8,q,<br>1H | 3.8,s,3H |
| 49 | 6.8–7.3,m,5H<br>8.2,dofd,1H<br>8.7,m,1H | 2.9,s,<br>6H | 8.7,m,<br>1H | 1.7,d,<br>3H | 4.9,q,<br>1H | 3.8,s,3H |
| 51 | 6.7–7.3,6H<br>7.7,s,1H | 1.3–<br>2.0,m,<br>2H<br>3.5–<br>4.0,m,<br>2H<br>0.6–<br>1.2,m,<br>3H | 9.3,s,<br>1H | 1.3–<br>2.0,m,<br>3H | 4.8,q,<br>1H | 3.5–4.0,<br>m,3H |
| 52 | 6.8–7.8,m,6H<br>8.5,s,1H | | 9.7,s,<br>1H | 1.6,d,<br>3H | 4.8,q,<br>1H | 1.2,t,3H<br>4.3,q,2H |
| 53 | 6.6–7.3,dofd,<br>4H<br>7.5–7.8,m,1H<br>8.2,dofd,1H<br>8.6,d,1H | 3.9,s,<br>3H | 7.5–<br>7.9,m,<br>1H | 1.6,d,<br>3H | 4.7,q,<br>1H | 3.7,s,3H |
| 55 | 6.8–7.5,m } 7H<br>8.0–8.5,m } 7H | | 3.6,s,<br>1H | 1.6,d,<br>3H | 4.8,q,<br>1H | 1.4,s,10H |
| 56 | 7.0,q,4H<br>7.1–8.0,m,3H | | 3.5,t,<br>1H<br>4.5,d,<br>2H | 1,5,d,<br>3H | 4.8,q,<br>1H | 3.8,s,3H |
| 58 | 6.7–7.7,m,6H<br>8.5,s,1H | | 9.7,s,<br>1H | 1.6,d,<br>3H | 4.5,q,<br>1H | 3.8,d,2H<br>2.5,s,1H |
| 59 | 6.5–8.2,m,7H | | 3.4,s,<br>3H | 1.3,d,<br>3H | 4.5,q,<br>1H | 3.8,d,2H<br>2.5,s,1H |
| 60 | 6.5–7.3,m,6H<br>8.4,s,1H | | 9.4,s,<br>1H | 1.1–<br>1.6,<br>3H | 4.3,q,<br>1H | 1.1–1.6,<br>m,3H<br>2.1,q,2H<br>4.0,d,2H |
| 63 | 6.8–7.6,m,6H<br>8.2,s,1H | | 9.6,s,<br>1H | 1.6,d,<br>3H | 4.8,q,<br>1H | 2.2,s,6H<br>1.5,t,2H<br>4.3,t,2H |
| 66 | 6.8–7.8,m,6H<br>8.1,d,1H | | 2.0,s,<br>3H | 1.6,d,<br>3H | 4.8,q,<br>1H | 1.2,t,3H<br>4.2,q,2H |
| 67 | 6.7–7.3,m,5H<br>7.7,dodfd,1H<br>8.0,d,1H | | 3.4,s,<br>3H | 1.6,d,<br>3H | 4.8,q,<br>1H | 1.2,t,3H<br>4.3,q,2H |

TABLE 2 PART A (Continued)

| Compound No. | PROTON CHEMICAL SHIFT δ IN PPM ($CDCl_3$) | | | | | |
|---|---|---|---|---|---|---|
| | Phenyl | A, D Groups | $R^1$ | $R^2$ | $R^3$ | W Group |
| 69 | 7.0–8.0,m,6H 8.7,s,1H | | 9.7,d, 1H | 1.7,d, 3H | 4.8,q, 1H | 1.2,t,6H 2.4–3.0,m,6H 4,4,t,2H |
| 71 | 6.9–7.6,m,6H 8.0,s,1H | 3.9,s, 3H | 9.4,s, 1H | 1.6,d, 3H | 4.7,q, 1H | 1.2,t,3H 4.2,q,2H |
| 72 | 6.8–7.8,m,6H 8.5,d,1H | | 9.8,s, 1H | 1.0–1.9,m, 3H | 4.8,q, 1H | 0.9,t,3H 4.2,t,2H 1.0–1.9,m,2H |
| 73 | 6.7–7.7,m,6H 8.5,s,1H | | 9.6,s, 1H | 1.6,d, 3H | 4.8,q, 1H | 2.1,s,3H 2.7,t,2H 4.3,t,2H |
| 74 | 6.7–8.0,m,6H 8.3,s,1H | | 6.7–8.0,m, 4H | 1.6,d, 3H | 4.7,q, 1H | 1.2,t,3H 4.2,q,2H |
| 78 | 6.7–7.6,m,5H 8.3,s,1H | | 9.5,s, 1H | 1.5,d, 3H | 4.6,q, 1H | 3.7,s,3H |
| 79 | 6.9–7.7,m,6H 8.5,s,1H | | 9.7,s, 1H | 1.7,s, 3H | 1.7,s, 3H | 1.2,t,3H 4.3,q,2H |

TABLE 2 PART B

| Compound No. | Melting Point °C | Compound No. | Melting Point °C |
|---|---|---|---|
| 20 | 144 | 50 | 105 |
| | | 54 | 102 |
| 28 | 110 | 57 | 81 |
| 29 | 120 | 62 | 94 |
| | | 64 | 138–140 |
| | | 65 | 156 |
| | | 68 | 98 |
| | | 70 | 84 |
| 37 | 110 | | |
| 40 | 122 | 75 | 90 |
| | | 76 | 94 |
| 42 | 122 | 77 | 84 |
| 44 | 199 | | |
| 48 | 104 | | |

TABLE 2 PART C

| Compound No. | Partial Mass Spectrum m/e (%) |
|---|---|
| 49 | $M^+$ 341(64), 254(100), 208(68) |

Example 21

Concentrated formulations of the compounds of the invention were prepared by:

a) in the case of oils and waxy solids, dissolving the compound in toluene containing 7% v/v "Teric" N13 ("Teric" is a Trade Mark and "Teric" N13, a product of ethoxylation of nonylphenol, is available from ICI Australia Limited) and 3% v/v "Kemmat" SC15B ("Kemmat" is a Trade Mark and "Kemmat" SC15B is a formulation of calcium dodecylbenzene sulfonate); or

b) in the case of crystalline solids, adding 5 parts by weight of the compound and 1 part by weight of "Dyapol" PT ("Dyapol" is a Trade Mark and "Dyapol" PT is an anionic suspending agent) to 94 parts by weight of an aqueous solution containing 0.25% v/v of "Teric" N8 (a product of ethoxylation of nonylphenol) and ball-milling the mixture to produce a stable suspension.

The emulsifiable concentrates and suspensions were then diluted with water to give an aqueous composition of the required concentration suitable for use in the evaluation of the pre-emergence and post-emergence herbicidal activity of the compounds of the invention.

Example 22

The pre-emergent herbicidal activity of the compounds of the invention formulated as described in Example 21 was assessed by the following procedure.

The seeds of the test species were sown in rows 2 cm deep in soil contained in seed boxes. The monocotyledonous plants and the dicotyledonous plants were sprayed with the required quantity of a

composition of the invention. Two duplicate seed boxes were prepared in the same manner but were not sprayed with a composition of the invention and were used for comparison purposes. All the boxes were placed in a glasshouse, lightly watered with an overhead spray to initiate germination and then sub-irrigated as required for optimum plant growth. After three weeks the boxes were removed from the glasshouse and the effect of the tratment was visually assessed. The results are presented in Table 3 where the damage to plants is rated on a scale of from 0 to 3 where 0 represents from 0 to 25% damage, 3 represents 75 to 99% kill and 3+ represents 100% kill.

A (–) means that no experiment was carried out.

The names of the test plants are as follows:

| | |
|---|---|
| Wh | Wheat |
| Ot | Wild Oats |
| Rg | Ryegrass |
| Jm | Japanese millet |
| P | Peas |
| Ip | Ipomea |
| Ms | Mustard |
| Sf | Sunflower. |

TABLE 3

PRE-EMERGENCE HERBICIDAL ACTIVITY

| Compound No. | Application Rate kg/ha | Test Plant | | | | | | | |
|---|---|---|---|---|---|---|---|---|---|
| | | Wh | Ot | Rg | Jm | P | Ip | Ms | Sf |
| 1 | 5 | 0 | 0 | 3 | 0 | 0 | 0 | 0 | 0 |
| 1 | 1 | 0 | 0 | 1 | 0 | 0 | 0 | 0 | 0 |
| 7 | 5 | 0 | 0 | 1 | 0 | 0 | 0 | 0 | 0 |
| 7 | 1 | 0 | 0 | 0 | 0 | 0 | 0 | 0 | 0 |
| 9 | 10 | 0 | 1 | 3 | 3 | 0 | 0 | 0 | 0 |
| 15 | 5 | 0 | 0 | 2 | 2 | 0 | 0 | 0 | 0 |
| 17 | 10 | 1 | – | 3+ | 3 | 0 | 0 | 0 | 0 |
| 18 | 5 | 1 | – | 3+ | 3 | 0 | 0 | 0 | 0 |
| 18 | 1 | 0 | – | 2 | 0 | 0 | 0 | 0 | 0 |
| 19 | 5 | 0 | 0 | 2 | 1 | 0 | 0 | 0 | 0 |
| 33 | 0.5 | 0 | 2 | 3 | 3+ | 0 | 0 | 0 | 0 |
| 54 | 5 | 0 | 2 | 3 | 3+ | 0 | 0 | 0 | 0 |
| 54 | 1 | 0 | 1 | 3 | 3 | 0 | 0 | 0 | 0 |
| 55 | 5 | 1 | 2 | 3+ | 3 | 0 | 0 | 0 | 0 |
| 55 | 1 | 0 | 1 | 3 | 0 | 0 | 0 | 0 | 0 |
| 56 | 5 | 3 | 2 | 3+ | 3+ | 0 | 0 | 0 | 0 |
| 56 | 1 | 0 | 1 | 3 | 3+ | 0 | 0 | 0 | 0 |
| 62 | 5 | 2 | 3+ | 3+ | 3+ | 0 | 0 | 0 | 0 |
| 62 | 1 | 0 | 2 | 3 | 3 | 0 | 0 | 0 | 0 |
| 63 | 5 | 0 | 1 | 3 | 3 | 0 | 0 | 0 | 0 |
| 64 | 5 | 0 | 3 | 2 | 0 | 0 | 0 | 0 | 0 |
| 65 | 5 | 2 | 3 | 3+ | 3+ | 0 | 0 | 0 | 0 |
| 65 | 1 | 0 | 1 | 3 | 2 | 0 | 0 | 0 | 0 |
| 67 | 5 | 0 | 1 | 2 | 1 | 0 | 0 | 0 | 0 |
| 69 | 5 | 2 | 3 | 3+ | 3+ | 0 | 0 | 0 | 0 |
| 69 | 1 | 0 | 2 | 3 | 2 | 0 | 0 | 0 | 0 |
| 70 | 5 | 3 | 3+ | 3+ | 3+ | 0 | 0 | 0 | 0 |
| 70 | 1 | 1 | 3 | 3 | 3 | 0 | 0 | 0 | 0 |

Example 23

The post-emergent herbicidal activity of the compounds of the invention formulated as described in Example 21 was assessed by the following procedure.

The seeds of the test species were sown in rows 2 cm deep in soil contained in seed boxes. The monocotyledonous plants and the dicotyledonous plants were sown in separate seed boxes in duplicate. The four seed boxes were placed in a glasshouse, lightly watered with an overhead spray to initiate germination and then sub-irrigated as required for optimum plant growth. After the plants had grown to a height of about 10 to 12.5 cm one box of each of the monocotyledonous plants and the dicotyledonous plants was removed from the glasshouse and sprayed with the required quantity of a composition of the invention. After spraying the boxes were returned to the glasshouse for further 3 weeks and the effects of the treatment was visually assessed by comparison with the untreated controls. The results are presented in Table 4 where the damage to plants is rated on a scale of from 0 to 3 where 0 represents 0 to 25% damage, 3 represents 75 to 99% kill and 3+ represents 100% kill. A dash (—) means that no experiment was carried out.

The names of the test plants are as follows:

| | |
|---|---|
| Wh | Wheat |
| Ot | Wild Oats |
| Rg | Ryegrass |
| Jm | Japanese millet |
| P | Peas |
| Ip | Ipomea |
| Ms | Mustard |
| Sf | Sunflower |

TABLE 4

POST-EMERGENCE HERBICIDAL ACTIVITY

| Compound No. | Application Rate kg/ha | Test Plant | | | | | | | |
|---|---|---|---|---|---|---|---|---|---|
| | | Wh | Ot | Rg | Jm | P | Ip | Ms | Sf |
| 1 | 5 | 1 | 3 | 3+ | 3+ | 0 | 0 | 0 | 0 |
| 1 | 1 | 0 | 0 | 3+ | 3+ | 0 | 0 | 0 | 0 |
| 7 | 5 | 0 | 0 | 3 | 3 | 0 | 0 | 0 | 0 |
| 7 | 1 | 0 | 0 | 1 | 3 | 0 | 0 | 0 | 0 |
| 8 | 5 | 0 | 0 | 0 | 3 | 2 | 3+ | 3+ | 2 |
| 9 | 10 | 2 | 2 | 3+ | 3+ | 0 | 3 | 3 | 0 |
| 9 | 5 | 2 | 3 | 3+ | 3+ | 0 | 0 | 0 | 0 |
| 9 | 1 | 0 | 0 | 1 | 3 | 0 | 0 | 1 | 0 |
| 12 | 5 | 2 | 3 | 3+ | 3+ | 0 | 0 | 0 | 0 |
| 15 | 5 | 3 | 3+ | 3+ | 3+ | 0 | 3+ | 3+ | 3+ |
| 15 | 1 | 1 | 2 | 3+ | 3+ | 0 | 0 | 1 | 0 |
| 17 | 10 | 3 | 3 | 3+ | 3+ | 0 | 0 | 0 | 0 |
| 18 | 5 | 3 | 1 | 3 | 3+ | 0 | 0 | 0 | 0 |
| 18 | 1 | 2 | 1 | 3 | 3 | 0 | 0 | 0 | 0 |
| 19 | 5 | 3 | 3 | 3 | 3+ | 0 | 0 | 0 | 0 |
| 19 | 1 | 0 | 1 | 2 | 3 | 0 | 0 | 0 | 0 |
| 27 | 5 | 2 | 2 | 3 | 3+ | 0 | 0 | 0 | 0 |
| 27 | 1 | 1 | – | 2 | 2 | 0 | 2 | 2 | 0 |
| 29 | 5 | 0 | 2 | 3 | 3 | 0 | 0 | 0 | 0 |
| 29 | 1 | 0 | 1 | 1 | 3 | 0 | 0 | 0 | 0 |
| 33 | 0.5 | 0 | 3 | 3 | 3+ | 0 | 0 | 0 | 0 |
| 54 | 5 | 3 | 3 | 3 | 3+ | 0 | 0 | 0 | 0 |
| 54 | 1 | 2 | 3 | 3+ | 3+ | 0 | 0 | 0 | 0 |
| 55 | 5 | 3 | 3+ | 3+ | 3+ | 0 | 0 | 0 | 0 |
| 55 | 1 | 1 | 3+ | 3 | 3+ | 0 | 0 | 0 | 0 |
| 56 | 5 | 3 | 3+ | 3+ | 3+ | 0 | 0 | 0 | 0 |
| 56 | 1 | 2 | 3+ | 3 | 3+ | 0 | 0 | 0 | 0 |

TABLE 4 (Continued)

| Compound No. | Application Rate kg/ha | Test Plant | | | | | | | |
|---|---|---|---|---|---|---|---|---|---|
| | | Wh | Ot | Rg | Jm | P | Ip | Ms | Sf |
| 58 | 5 | 0 | 3 | 0 | 3+ | 0 | 0 | 0 | 0 |
| 59 | 5 | 0 | 3 | 2 | 1 | 0 | 0 | 0 | 0 |
| 60 | 5 | 3+ | 3+ | 3+ | 3+ | 0 | 0 | 0 | 0 |
| 60 | 1 | 2 | 2 | 1 | 3+ | 0 | 0 | 0 | 0 |
| 62 | 5 | 3+ | 3+ | 3+ | 3+ | 0 | 0 | 0 | 0 |
| 62 | 1 | 3 | 3+ | 3 | 3+ | 0 | 0 | 0 | 0 |
| 63 | 5 | 3+ | 3+ | 3+ | 3+ | 0 | 0 | 0 | 0 |
| 63 | 1 | 2 | 2 | 3 | 3 | 0 | 0 | 0 | 0 |
| 64 | 5 | 3 | 3+ | 3+ | 3+ | 0 | 0 | 0 | 0 |
| 64 | 1 | 2 | 2 | 3 | 3+ | 0 | 0 | 0 | 0 |
| 65 | 5 | 3+ | 3+ | 3+ | 3+ | 0 | 0 | 0 | 0 |
| 65 | 1 | 3+ | 3 | 3+ | 3+ | 0 | 0 | 0 | 0 |
| 67 | 5 | 3 | 2 | 2 | 3+ | 0 | 0 | 0 | 0 |
| 67 | 1 | 3 | 2 | 2 | 3+ | 0 | 0 | 0 | 0 |
| 69 | 5 | 3+ | 3+ | 3+ | 3+ | 0 | 0 | 0 | 0 |
| 69 | 1 | 3 | 3 | 3+ | 3+ | 0 | 0 | 0 | 0 |
| 70 | 5 | 3+ | 3+ | 3+ | 3+ | 0 | 0 | 0 | 0 |
| 70 | 1 | 3+ | 3+ | 3+ | 3+ | 0 | 0 | 0 | 0 |
| 77 | 5 | 0 | 2 | 3 | 3 | 0 | 0 | 0 | 0 |

## Example 24

The compounds were formulated for test by mixing an appropriate amount with 5 ml of an emulsion prepared by diluting 160 ml of a solution containing 21.8 g per litre of "Span" 80 and 78.2 g per litre of "Tween" 20 in methylcyclohexanone to 500 ml with water. "Span" 80 is a Trade Mark for a surface-active agent comprising sorbitan monolaurate. "Tween" 20 is a Trade Mark for a surface-active agent comprising a condensate of sorbitan monolaurate with 20 molar proportions of ethylene oxide. Each 5 ml emulsion containing a test compound was then diluted to 40 ml with water and sprayed on to young pot plants (post-emergence test) of the species named in Table 5 below. Damage to test plants was assessed after 14 days on a scale of 0 to 5 where 0 is 0 to 20% damage and 5 is complete kill. In a test for pre-emergence herbicidal activity, seeds of the test plants were sown in a shallow slit formed in the surface of soil in fibre trays. The surface was then levelled and sprayed, and fresh soil then spread thinly over the sprayed surface. Assessment of herbicidal damage was carried out after 21 days using the same scale of 0 to 5 as the post-emergence test. In both cases the degree of herbicidal damage was assessed by comparison with untreated control plants. The results are given in Table 5 below. A dash (—) means that no experiment was carried out.

The names of the test plants were as follows:

| | |
|---|---|
| Sb | Sugar beet |
| Rp | Rape |
| Ct | Cotton |
| Sy | Soy bean |
| Mz | Maize |
| Ww | Winter wheat |
| Rc | Rice |
| Sn | *Senecio vulgaris* |
| Ip | *Ipeomea purpurea* |
| Am | *Amaranthus retroflexus* |
| Pi | *Polygonum aviculare* |
| Ca | *Chenopodium album* |
| Po | *Portulaca oleracea* |
| Xa | *Xanthium pensylvanicum* |
| Ab | *Abutilon theophrasti* |
| Cv | *Convolvulus arvensis* |
| Ot | Cultivated oats and wild oats *(Avena fatua)* Wild oats are used in the post-emergence test and cultivated oats in the pre-emergence test |
| Dg | *Digitaria sanguinalis* |
| Pu | *Poa annua* |
| St | *Setaria viridis* |
| Ec | *Echinochloa crus-galli* |
| Sh | *Sorghum halepense* |
| Ag | *Agropyron repens* |
| Cn | *Cyperus rotundus* |

TABLE 5 – PART A

| Compound No. | Application Method Rate (kg/ha) | Test Plant | | | | | | | | | | | |
|---|---|---|---|---|---|---|---|---|---|---|---|---|---|
| | | Sb | Rp | Ct | Sy | Mz | Ww | Rc | Sn | Ip | Am | Pi | Ca |
| 1 | PRE 2.0 | 0 | 2 | 0 | 2 | 0 | 0 | 0 | 0 | 0 | – | – | – |
| 1 | POST 2.0 | 3 | 2 | 1 | 1 | 5 | 3 | 0 | 1 | 0 | 2 | 0 | 3 |
| 6 | PRE 2.0 | 2 | 1 | 1 | 1 | 0 | 0 | 0 | 0 | – | – | – | – |
| 6 | POST 2.0 | 1 | 0 | 0 | 0 | 2 | 0 | 0 | 1 | 0 | 2 | 0 | 0 |
| 9 | PRE 2.0 | 0 | 0 | 0 | 1 | 2 | 0 | 0 | 0 | 0 | – | – | – |
| 9 | POST 2.0 | 0 | 2 | 2 | 2 | 4 | 2 | 2 | 0 | 0 | 2 | 0 | 1 |
| 12 | PRE 2.0 | 0 | 1 | 0 | 0 | 1 | 0 | 1 | 0 | 0 | 0 | – | – |
| 12 | POST 2.0 | 1 | 0 | 0 | 0 | 2 | 0 | 0 | 0 | 0 | – | – | – |
| 29 | PRE 2.0 | 0 | 0 | 0 | 0 | 1 | 1 | 0 | 3 | 2 | 0 | 0 | – |
| 29 | POST 2.0 | 0 | 0 | 0 | 0 | 4 | 3 | 0 | 0 | 0 | 1 | 0 | 0 |
| 56 | PRE 2.0 | 0 | 0 | 0 | 0 | 3 | 4 | 4 | 0 | 0 | 0 | 0 | 0 |
| 56 | POST 2.0 | 3 | 0 | 0 | 0 | 4 | 4 | 4 | – | 0 | 0 | 0 | 0 |
| 77 | PRE 2.0 | 0 | 0 | 0 | 0 | 0 | 0 | 0 | 5 | 0 | 0 | 0 | – |
| 77 | POST 2.0 | 2 | 0 | 0 | 0 | 0 | 0 | 0 | 3 | 0 | 0 | 0 | 0 |
| 78 | PRE 2.0 | 0 | 0 | 0 | 0 | 3 | 4 | 4 | 0 | 0 | 0 | 0 | 0 |
| 78 | POST 2.0 | 3 | 0 | 0 | 0 | 4 | 4 | 4 | – | 0 | 0 | 0 | 0 |

TABLE 5 – PART B

| Compound No. | Application Method Rate (kg/ha) | | Test Plant | | | | | | | | | | | |
|---|---|---|---|---|---|---|---|---|---|---|---|---|---|---|
| | | | Po | Xa | Ab | Cv | Ot | Dg | Pu | St | Ex | Sh | Ag | Cn |
| 1 | PRE | 2.0 | 3 | 0 | 2 | – | 0 | 2 | – | 0 | 0 | – | 1 | 0 |
| 1 | POST | 2.0 | 0 | 1 | 1 | 0 | 0 | 1 | 0 | 4 | 4 | 3 | 0 | 0 |
| 6 | PRE | 2.0 | 0 | 0 | 0 | – | 0 | 0 | – | 0 | 0 | – | – | 0 |
| 6 | POST | 2.0 | 1 | 0 | 0 | 0 | 0 | 0 | 0 | 3 | 2 | 3 | 0 | 0 |
| 9 | PRE | 2.0 | 0 | 0 | 1 | – | 0 | 0 | – | 0 | 0 | – | 0 | 0 |
| 9 | POST | 2.0 | 0 | 0 | 0 | 0 | 2 | 2 | 0 | 4 | 4 | 5 | 1 | 0 |
| 12 | PRE | 2.0 | 0 | 0 | 0 | – | 0 | 1 | 0 | 0 | 0 | 2 | 0 | 0 |
| 12 | POST | 2.0 | 1 | 0 | 0 | 0 | 0 | 3 | 0 | 4 | 3 | 1 | 0 | 0 |
| 29 | PRE | 2.0 | 0 | 0 | 0 | – | 2 | 2 | 0 | 5 | 1 | 0 | 0 | 0 |
| 29 | POST | 2.0 | 0 | 0 | 0 | 0 | 4 | 4 | 0 | 4 | 4 | 4 | 0 | 0 |
| 56 | PRE | 2.0 | 0 | 0 | 0 | – | 3 | 5 | 3 | 5 | 5 | 4 | 4 | 0 |
| 56 | POST | 2.0 | 0 | 0 | 0 | 1 | 5 | 4 | 2 | 5 | 4 | 5 | 3 | 0 |
| 77 | PRE | 2.0 | 0 | 0 | 0 | – | 1 | 0 | 4 | 2 | 0 | 0 | – | 0 |
| 77 | POST | 2.0 | 0 | 2 | 0 | 0 | 0 | 0 | 0 | 2 | 0 | 4 | 0 | 0 |
| 78 | PRE | 2.0 | 0 | 0 | 0 | – | 0 | 4 | 0 | 0 | 0 | 0 | 0 | 0 |
| 78 | POST | 2.0 | 0 | 0 | 0 | 0 | 0 | 1 | 0 | 3 | 1 | 3 | 0 | 0 |

Example 25

This Example illustrates the selective herbicidal activity of compounds of the invention.

The seeds of the test species were sprinkled onto the surface of soil in seed boxes and covered with a thin layer of sand. The boxes were lightly watered with an overhead spray and placed in a glasshouse for one week to permit germination of the seeds and plant growth to a height of 4 to 5 inches. The boxes were then removed from the glasshouse and sprayed with a composition of the invention prepared as described in Example 30. For comparison purposes at least one box containing one week old seedlings was sprayed lightly with water only. After spraying the boxes were returned to the glasshouse for a further 29 days after which the effect of the treatment was visually assessed. The tests were carried out in triplicate and the results, expressed as percentage damage, are recorded in Table 6. To assess the safety of the test compounds on the test crop plant species the crop plant species were tested at an application rate ten times higher than that used for the weed plant species.

The names of the test plants were as follows:

| | |
|---|---|
| Ct | Cotton |
| Sb | Soyabean |
| Ot | Wild Oats |
| Rg | Annual Rye Grass |
| Jm | Japanese Millet |
| St | *Setaria aviceps* |
| Sh | *Sorghum halepense* |

TABLE 6

| Compound No. | Crops | | | Weeds | | | | | |
|---|---|---|---|---|---|---|---|---|---|
| | Rate kg/ha | Test Plant | | Rate kg/ha | Test Plant | | | | |
| | | Ct | Sb | | Ot | Rg | Jm | St | SH |
| | 1.5 | 10 | 15 | 0.15 | 50 | 73 | 95 | 38 | 100 |

Example 26

This Example illustrates the selective herbicidal activity of compounds of the invention.

The compounds were formulated for test by mixing an appropriate amount with 5 ml of an emulsion prepared by diluting 160 ml of a solution containing 21.8 g per litre of "Span" 80 and 78.2 g per litre of "Tween" 20 in methylcyclohexanone to 500 ml with water. "Span" 80 is a Trade Mark for a surface-active agent comprising sorbitan monolaurate. "Tween" 20 is a Trade Mark for a surface-active agent comprising a condensate of sorbitan monolaurate with 20 molar proportions of ethylene oxide. Each 5 ml emulsion containing a test compound was then diluted to 40 ml with water and sprayed on to young pot plants (post-emergence test) of the species named in Table 7 below. Damage to test plants was assessed after 20 days on a scale of 0 to 9 where 0 is 0 to 10% and 9 is 90 to 100% kill. The tests were carried out in triplicate and the mean results are recorded in Table 7 below. The names of the test plants were as follows:

| | |
|---|---|
| Sy | Soyabean (Amsoy) |
| Ct | Cotton (Deltapine) |
| Ec | *Echinochloa crus-galli* |
| Sh | *Sorghum halepense* |
| St | *Setaria viridis* |
| Dg | *Digitaria sanguinalis* |

TABLE 7

| Compound No. | Rate kg/ha | Test Plants | | | | | |
|---|---|---|---|---|---|---|---|
| | | Crops | | Weeds | | | |
| | | Sy | Ct | Ec | Sh | St | Dg |
| 32 | 0.08 | 0 | 0 | 9 | 8 | 7 | 4 |
| 33 | 0.08 | 0 | 0 | 5 | 9 | 6 | 5 |
| 38 | 0.08 | 0 | 0 | 9 | 9 | 9 | 9 |

## Claims

1. A compound of formula II

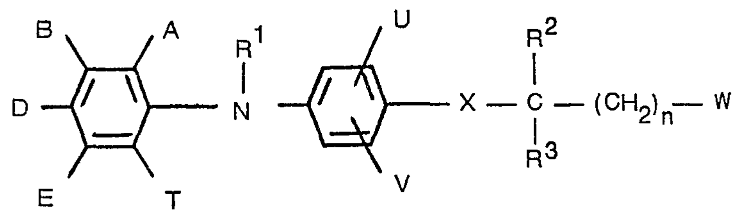

II.

or a salt thereof wherein:

A, B, D, E and T are independently chosen from the group consisting of hydrogen, halogen, nitro, cyano, amino, formyl, $C_1$ to $C_6$ alkoxy, $C_1$ to $C_6$ haloalkyl, $C_1$ to $C_6$ haloalkoxy, ($C_1$ to $C_6$ alkoxy)carbonyl, $C_1$ to $C_6$ haloalkylthio, $C_1$ to $C_6$ haloalkylsulfinyl, $C_1$ to $C_6$ alkylsulfonyl, $C_1$ to $C_6$ haloalkylsulfonyl, sulfo, $C_1$ to $C_6$ alkoxysulfonyl, N,N-di($C_1$ to $C_6$ alkyl)carbamoyl, sulfamoyl and N,N-di($C_1$ to $C_6$ alkyl)sulfamoyl;

U and V are independently chosen from hydrogen, halogen, and nitro;

$R^1$ is chosen from the group consisting of hydrogen, $C_1$ to $C_6$ alkyl, $C_2$ to $C_6$ alkenyl, $C_2$ to $C_6$ alkynyl, $C_2$ to $C_6$ alkanoyl, benzyl, benzoyl and halobenzoyl;

$R^2$ and $R^3$ are independently chosen from hydrogen and $C_1$ to $C_6$ alkyl;

W is chosen from the groups

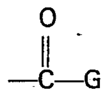 and $—CH_2Z$

wherein: G is chosen from the group consisting of hydroxy, mercapto, $C_1$ to $C_{10}$ alkoxy, $C_2$ to $C_{10}$ alkenyloxy, $C_2$ to $C_{10}$ alkynyloxy, $C_3$ to $C_7$ cycloalkoxy, $C_1$ to $C_{10}$ alkylthio, benzyloxy, N,N-di($C_1$ to $C_6$ alkyl)amino, $C_1$ to $C_{10}$ alkoxy substituted with a substituent chosen from halogen, amino, ammonio, N-($C_1$ to $C_6$ alkyl)amino, N,N-di($C_1$ to $C_6$ alkyl)amino, N,N,N-tri-($C_1$ to $C_6$ alkyl)ammonio, $C_1$ to $C_6$ alkoxy, $C_1$ to $C_6$ alkylthio, morpholino and 2-pyridyl, and the group OM wherein M is an alkali metal or alkaline earth metal ion; Z is chosen from the group consisting of hydroxy and $C_1$ to $C_6$ alkoxy;

X is oxygen; and

n is 0 or 2;

and provided that when:

B, E, T, U, V and $R^3$ are hydrogen;

D is trifluoromethyl or halogen;

$R^1$ is hydrogen, $C_1$ to $C_6$ alkyl, $C_2$ to $C_6$ alkenyl or benzyl;

$R^2$ is $C_1$ to $C_6$ alkyl;

W is the group

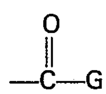

wherein G is chosen from hydroxy, mercapto, $C_1$ to $C_{10}$ alkoxy, $C_1$ to $C_{10}$ alkylthio or the group OM wherein M is analkali metal ion:

X is oxygen; and

n is 0 or 2; then

A is not nitro.

2. A compound according to claim 1 wherein:

A is nitro;

D is chosen from the group consisting of hydrogen, nitro, cyano, $C_1$ to $C_6$ alkyl; $C_1$ to $C_6$ alkoxy and $C_1$ to $C_6$ haloalkoxy;

$R^1$ is chosen from the group consisting of hydrogen, $C_1$ to $C_6$ alkyl, $C_2$ to $C_6$ alkenyl, $C_2$ to $C_6$alkynyl and benzyl;

$R^2$ is chosen from hydrogen and $C_1$ to $C_6$ alkyl;

B, E, T, U, V and $R^3$ are hydrogen;

W is chosen from the groups

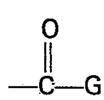 and $—CH_2Z$

wherein: G is chosen from the group consisting of hydroxy, mercapto, $C_1$ to $C_{10}$ alkoxy, $C_2$ to $C_{10}$ alkenyloxy, $C_2$ to $C_{10}$ alkynyloxy, $C_3$ to $C_7$ cycloalkoxy, $C_1$ to $C_{10}$ alkylthio, benzyloxy, N,N-di($C_1$ to $C_6$ alkyl)amino, the group OM wherein M is an alkali metal or alkaline earth metal ion, and the group $C_1$ to $C_{10}$ alkoxy substituted with a substituent chosen from $C_1$ to $C_6$ alkoxy, $C_1$ to $C_6$ alkylthio, halogen, N,N-di-($C_1$ to $C_6$ alkyl)amino, N,N,N-tri($C_1$ to $C_6$)ammonio and morpholino; and Z is chosen from the group consisting of hydroxy and $C_1$ to $C_6$ alkoxy;

X is oxygen; and

n is 0.

3. A compound according to claim 1 wherein:

A is chosen from the group consisting of hydrogen, halogen, $C_1$ to $C_6$ haloalkyl, cyano and amino;

D is chosen from the group consisting of hydrogen, nitro, cyano, halogen, $C_1$ to $C_6$ alkyl, $C_1$ to $C_6$ haloalkyl, $C_1$ to $C_6$ alkoxy and $C_1$ to $C_6$ haloalkoxy;

$R^1$ is chosen from the group consisting of hydrogen, $C_1$ to $C_6$ alkyl, $C_2$ to $C_6$ alkenyl, $C_2$ to $C_6$ alkynyl and benzyl;

$R^2$ is chosen from hydrogen and $C_1$ to $C_6$ alkyl;

B, E, T, U, V and $R^3$ are hydrogen;

W is chosen from the groups

$$-\overset{\overset{\displaystyle O}{\|}}{C}-G \text{ and } -CH_2Z$$

wherein: G is chosen from the group consisting of hydroxy, mercapto, $C_1$ to $C_{10}$ alkoxy, $C_2$ to $C_{10}$ alkenyloxy, $C_2$ to $C_{10}$ alkynyloxy, cyclohexyloxy, $C_2$ to $C_{10}$ alkylthio, benzyloxy, N,N-di($C_1$ to $C_6$ alkyl)amino, the group OM wherein M is an alkali metal or alkaline earth metal ion, and the group $C_1$ to $C_{10}$ alkoxy substituted with a substituent chosen from $C_1$ to $C_6$ alkoxy, $C_1$ to $C_6$ alkylthio, halogen, N,N-di($C_1$ to $C_6$ alkyl)amino, N,N,N-tri($C_1$ to $C_6$ alkyl)ammonio, and morpholino; and Z is chosen from the group consisting of hydroxy and $C_1$ to $C_6$ alkoxy;

X is oxygen; and

n is 0.

4. A compound according to claim 1 wherein:

A is nitro;

D is chosen from $C_1$ to $C_6$ haloalkyl and halogen;

$R^1$ is chosen from the group consisting of hydrogen, $C_1$ to $C_6$ alkyl, $C_2$ to $C_6$ alkenyl and benzyl;

$R^2$ is chosen from hydrogen and $C_1$ to $C_6$ alkyl;

B, E, T, U, V and $R^3$ are hydrogen;

W is chosen from the groups

$$-\overset{\overset{\displaystyle O}{\|}}{C}-G \text{ and } -CH_2Z$$

wherein G is chosen from the group consisting of $C_2$ to $C_{10}$ alkenyloxy, $C_2$ to $C_{10}$ alkynyloxy, $C_3$ to $C_7$ cycloalkoxy, benzyloxy, N,N-di($C_1$ to $C_6$ alkyl)amino, $C_1$ to $C_{10}$ alkoxy substituted with a substituent chosen from $C_1$ to $C_6$ alkoxy, $C_1$ to $C_6$ alkylthio, halogen, N,N-di($C_1$ to $C_6$ alkyl)amino, N,N,N-tri($C_1$ to $C_6$ alkyl)ammonio and morpholino; and Z is chosen from the group consisting of hydroxy and $C_1$ to $C_6$ alkoxy;

X is oxygen; and

n is 0.

5. A compound according to claim 1 wherein:

A is nitro;

D is chosen from $C_1$ to $C_6$ haloalkyl and halogen;

$R^1$ is $C_2$ to $C_6$ alkynyl;

$R^2$ is chosen from hydrogen and $C_1$ to $C_6$ alkyl;

B, E, T, U, V and $R^3$ are hydrogen;

W is chosen from the groups

$$-\overset{\overset{\displaystyle O}{\|}}{C}-G \text{ and } -CH_2Z$$

wherein: G is chosen from the group consisting of hydroxy, mercapto, $C_1$ to $C_6$ alkoxy, $C_2$ to $C_{10}$ alkenyloxy $C_2$ to $C_{10}$ alkynyloxy, $C_3$ to $C_7$ cycloalkoxy, $C_1$ to $C_{10}$ alkylthio, benzyloxy, N,N-di($C_1$ to $C_6$ alkyl)amino, the group OM wherein M is an alkali metal or alkaline earth metal ion, and the group $C_1$ to $C_6$ alkoxy substituted with a substituent chosen from $C_1$ to $C_6$ alkoxy, $C_1$ to $C_6$ alkylthio, halogen, N,N-di($C_1$ to $C_6$ alkyl)amino, N,N,N-tri($C_1$ to $C_6$ alkyl)ammonio and morpholino; and Z is chosen from the group consisting of hydroxy and $C_1$ to $C_6$ alkoxy;

X is oxygen; and

n is 0.

6. A compound according to claim 1 or 2 wherein:

A is nitro;

D is chosen from the group consisting of nitro, cyano, methyl, ethyl, methoxy and ethoxy;

$R^1$ is chosen from the group consisting of hydrogen, methyl, ethyl, n-propyl, n-butyl, allyl, propargyl and benzyl;

$R^2$ is methyl;

B, E, T, U, V and $R^3$ are hydrogen;

W is chosen from the groups

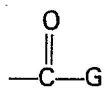 and $—CH_2Z$

wherein: G is chosen from the group consisting of hydroxy, $C_1$ to $C_6$ alkoxy, allyloxy, propargyloxy, $C_1$ to $C_4$ alkylthio, cyclohexyloxy, benzyloxy, N,N-dimethylamino, N,N-diethylamino, 2-haloethoxy, 2-($C_1$ to $C_6$ alkylthio)ethoxy, 2-(morpholino)ethoxy, 2-[N,N-di($C_1$ to $C_6$ alkyl)amino]ethoxy, 2-[N,N,N-tri($C_1$ to $C_6$ alkyl)ammonio]ethoxy and the groups OM wherein M is sodium or potassium; and Z is chosen from the group consisting of hydroxy and $C_1$ to $C_6$ alkoxy.

X is oxygen; and

n is 0.

7. A compound according to claim 1or 3 wherein:

A is chosen from the group consisting of hydrogen, fluoro, chloro, bromo, trifluoromethyl and amino;

D is chosen from the group consisting of fluoro, chloro, bromo, nitro, and trifluoromethyl;

$R^1$ is chosen from the group consisting of hydrogen, methyl, ethyl, n-propyl, n-butyl, allyl, propargyl and benzyl;

$R^2$ is methyl;

B, E, T, U, V and $R^3$ are hydrogen;

W is chosen from the groups

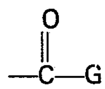 and $—CH_2Z$

wherein: G is chosen from the group consisting of hydroxy, $C_1$ to $C_6$ alkoxy, allyloxy, propargyloxy, cyclohexyloxy, benzyloxy, N,N-dimethylamino, N,N-diethylamino, 2-haloethoxy, 2-($C_1$ to $C_6$ alkylthio)-ethoxy, 2-(morpholino)ethoxy, 2-[N,N-di($C_1$ to $C_6$)amino]ethoxy, 2-[N,N,N-tri($C_1$ to $C_6$ alkyl)ammonio]-ethoxy, and the group OM wherein M is sodium or potassium; and Z is chosen from the group consisting of hydroxy and $C_1$ to $C_6$ alkoxy.

X is oxygen; and

n is 0.

8. A compound according to claim 1 or 4 wherein:

A is nitro;

D is chosen from the group consisting of fluoro, chloro, bromo and trifluoromethyl;

$R^1$ is chosen from the group consisting of hydrogen, methyl, ethyl, n-propyl, n-butyl, allyl and benzyl;

$R^2$ is methyl;

B, E, T, U, V and $R^3$ are hydrogen;

W is chosen from the groups

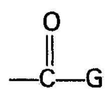 and $—CH_2Z$

wherein: G is chosen from the group consisting of allyloxy, propargyloxy, cyclohexyloxy, benzyloxy, N,N-dimethylamino, N,N-diethylamino, 2-haloethoxy, 2-($C_1$ to $C_6$ alkylthio)ethoxy, 2-(morpholino)ethoxy, 2-[N,N-di($C_1$ to $C_6$ alkyl)amino]ethoxy and 2-[N,N,N-tri($C_1$ to $C_6$ alkyl)ammonio]ethoxy; and Z is chosen from the group consisting of hydroxy, and $C_1$ to $C_6$ alkoxy;

X is oxygen; and

n is 0.

9. A compound according to claim 1 or 5 wherein:

A is nitro;

D is chosen from the group consisting of fluoro, chloro, bromo and trifluoromethyl;

$R^1$ is propargyl;

$R^2$ is methyl;

B, E, T, U, V and $R^3$ are hydrogen;

W is chosen from the groups

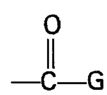 and $—CH_2Z$

wherein: G is chosen from the group consisting of hydroxy, $C_1$ to $C_6$ alkoxy, allyloxy, propargyloxy, cyclohexyloxy, benzyloxy, N,N-dimethylamino, N,N-diethylamino, 2-haloethoxy, 2-($C_1$ to $C_6$ alkylthio)-

ethoxy, 2-(morpholino)ethoxy, 2-[N,N-di($C_1$ to $C_6$ alkyl)amino]ethoxy, 2-[N,N,N-tri($C_1$ to $C_6$ alkyl)ammonio]ethoxy and the group OM wherein M is sodium or potassium; and Z is chosen from the group consisting of hydroxy and $C_1$ to $C_6$ alkoxy.

X is oxygen; and

n is 0.

10. A compound according to any one of claims 1, 2 and 6 selected from the group consisting of:
methyl 2-[4-(2,4-dinitroanilino)phenoxy]propionate;
methyl 2-[4-(4-methoxy-2-nitroanilino)phenoxy]propionate;
methyl 2-[4-(4-cyano-2-nitroanilino)phenoxy]propionate; and
ethyl 2-[4-(4-cyano-N-methyl-2-nitroanilino)phenoxy]propionate.

11. A compound according to any one of claims 1, 3 and 7 selected from the group consisting of:
methyl 2-[4-(2,4-dichloro-N-methylanilino) phenoxy]propionate;
methyl 2-[4-(2,4-dichloroanilino)phenoxy]propionate; and
methyl 2-[4-(4-nitroanilinophenoxy]propionate.

12. A compound according to any one of claims 1, 4 and 8 wherein:

A is nitro;

D is chosen from chloro and trifluoromethyl;

$R^1$ is chosen from hydrogen and methyl;

$R^2$ is methyl;

B, E, T, U, V and $R^3$ are hydrogen;

W is chosen from the groups:

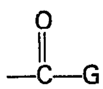

wherein G is chosen from the group consisting of allyloxy, propargyloxy, cyclohexyloxy, 2-(N,N-dimethylamino)ethoxy, 2-(N,N-diethylamino)ethoxy, 2-(N,N,N-trimethylammonio)ethoxy iodide and 2-bromoethoxy; and —$CH_2$Z wherein Z is ethoxy;

X is oxygen; and

n is 0.

13. A compound according to any one of claims 1, 5 and 9 which is:
methyl 2-[4-(2-nitro-N-propargyl-4-trifluoromethylanilino)-phenoxy]propionate.

14. A herbicidal composition comprising as active ingredient a compound as defined according to any one of claims 1 to 13 inclusive and a carrier therefor.

15. A composition according to claim 14 wherein said composition is in the form of a liquid and comprises a surface active agent.

16. A composition according to claim 14 wherein said composition is in the form of a powder.

17. A dilute composition according to any one of claims 14 to 16 inclusive which comprises from 0.01 to 2% by weight of active ingredient.

18. A concentrated composition according to any one of claims 14 to 16 inclusive which comprises from 20 to 90% by weight of active ingredient.

19. A process for severely damaging or killing unwanted plants which process comprises applying to said plants, or to the growth medium of said plants, an effective amount of a compound as defined according to any one of claims 1 to 13 inclusive or an effective amount of a composition as defined according to any one of claims 14 to 18 inclusive.

20. A process for selectively controlling the growth on monocotyledonous weeds in dicotyledonous crops which process comprises applying to said crop, or to the growth medium of said crop, a compound as defined according to any one of claims 1 to 13 inclusive or a composition as defined according to any one of claims 14 to 18 inclusive in an amount sufficient to severely damage or kill the weeds but insufficient to substantially damage the crop.

21. A process according to claim 19 or 20 wherein the compound is applied at a rate in the range from 0.005 to 20 kilograms per hectare.

22. A process according to claim 21 wherein the rate is in the range from 0.05 to 5 kilograms per hectare.

23. A process for the synthesis of a compound of formula I as defined according to any one of claims 1 to 13 inclusive which process comprises the condensation, in the presence of an alkaline material, of a phenol or thiophenol of formula IX with a compound of formula X, wherein hal is chlorine, bromine or iodine.

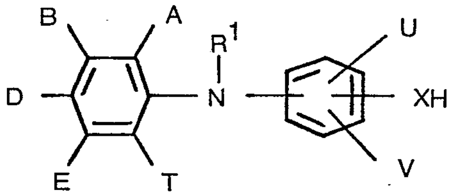

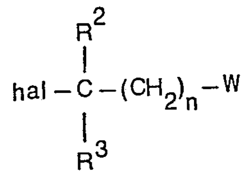

IX

24. A process for the synthesis of a compound of formula I as defined according to any one of claims 1 to 13 inclusive which process comprises the condensation of a compound of formula V, wherein L is a leaving group, with an aniline of formula VI.

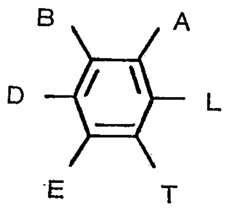

V

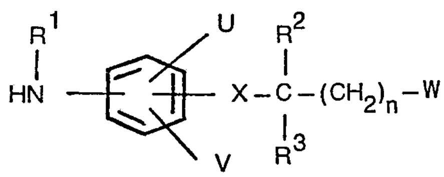

VI

25. A process for the synthesis of a compound of formula I as defined according to any one of claims 1 to 13 inclusive which process comprises the following steps in sequence:

a) the condensation of a compound of formula V, wherein L is a leaving group, with an aniline of formula VII, wherein Q is hydroxy, mercapto, $C_1$ to $C_6$ alkoxy, or $C_1$ to $C_6$ alkylthio, to give a compound of formula VIII:

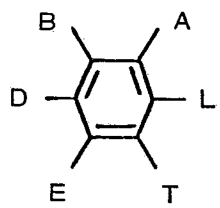

V

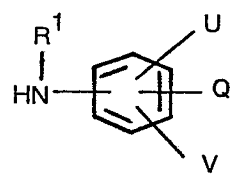

VII

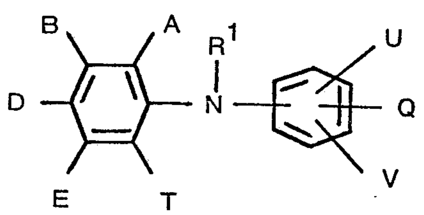

VIII

b) the dealkylation of the compound of formula VIII, wherein Q is $C_1$ to $C_6$ alkoxy or $C_1$ to $C_6$ alkylthio, to give a compound of formula IX; and

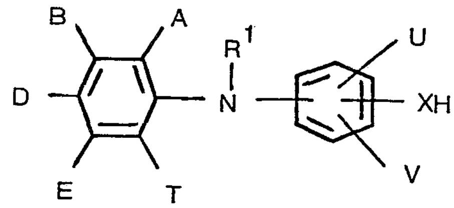

IX

c) the condensation, in the presence of an alkaline material, of a compound of formula IX obtained from step a) or step b) above with a compound of formula X wherein hal is chlorine, bromine or iodine.

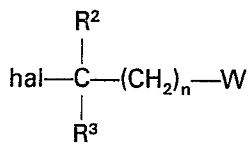

X

**Revendications**

1. Un composé de formule II

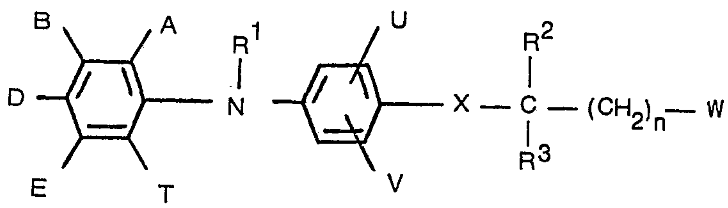

II

ou'un sel de ce composé, formule dans laquelle:

A, B, D, E et T sont choisis, indépendamment, entre l'hydrogène, un halogène, un groupe nitro, cyano, amino, formyle, alkyle en $C_1$ à $C_6$, alkoxy en $C_1$ à $C_6$, halogénalkyle en $C_1$ à $C_6$ halogénalkoxy en $C_1$ à $C_6$, (alkoxy en $C_1$ à $C_6$)-carbonyle, halogénalkylthio en $C_1$ à $C_6$, halogénalkylsulfinyle en $C_1$ à $C_6$, alkylsulfonyle en $C_1$ à $C_6$, halogénalkylsulfonyle en $C_1$ à $C_6$, sulfo, alkoxysulfonyle en $C_1$ à $C_6$, N,N-di-(alkyle en $C_1$ à $C_6$)-carbamoyle, sulfamoyle et N,N-di-(alkyle en $C_1$ à $C_6$-sulfamoyle;

U et V sont choisis, indépendamment, entre l'hydrogène, un halogène et le groupe nitro;

$R^1$ est choisi dans le groupe comprenant l'hydrogène, un radical alkyle en $C_1$ à $C_6$, alcényle en $C_1$ à $C_6$, alcynyle en $C_2$ à $C_6$, alcanoyle en $C_2$ à $C_6$, benzyle, benzoyle et halogénobenzoyle;

$R^2$ et $R^3$ sont choisis, indépendamment, entre l'hydrogène et un radical alkyle en $C_1$ à $C_6$;

W est un choisi entre

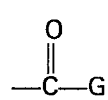 et $-CH_2Z$,

où: G est choisi entre un radical hydroxy, mercapto, alkoxy en $C_1$ à $C_{10}$, alcényloxy en $C_2$ à $C_{10}$, alcynyloxy en $C_2$ à $C_{10}$, cycloalkoxy en $C_3$ à $C_7$, alkylthio en $C_1$ à $C_{10}$, benzyloxy, N,N-di-(alkyle en $C_1$ à $C_6$)-amino, alkoxy en $C_1$ à $C_{10}$ substitué avec un substituant choisi entre un halogène, un groupe amino, ammonio, N-(alkyle en $C_1$ à $C_6$)-amino, N,N-di-(alkyle en $C_1$ à $C_6$)-amino, N,N,N-tri-(alkyle en $C_1$ à $C_6$)-ammonio, alkoxy en $C_1$ à $C_6$, alkylthio en $C_1$ à $C_6$, morpholino et 2-pyridyle et le groupe OM dans lequel M est un ion de métal alcalin ou de métal alcalino-terreux; Z est choisi entre un groupe hydroxy et un groupe alkoxy en $C_1$ à $C_6$;

X est l'oxygène; et

*n* est égal à 0 ou 2;

à condition que lorsque:

B, E, T, U, V et $R^3$ représentent l'hydrogène;

D est le groupe trifluorométhyle ou un halogène;

$R^1$ est l'hydrogène, un groupe alkyle en $C_1$ à $C_6$, un groupe alcényle en $C_2$ à $C_6$ ou le groupe benzyle;

$R^2$ est un groupe alkyle en $C_1$ à $C_6$;

W est le groupe

$$-\overset{\overset{\displaystyle O}{\|}}{C}-G,$$

dans lequel G est choisi entre un radical hydroxy, mercapto, alkoxy en $C_1$ à $C_{10}$, alkylthio en $C_1$ à $C_{10}$ ou le groupe OM, dans lequel M est un ion de métal alcalin,

X est l'oxygène; et

*n* est égal à 0 ou à 2;

A ne soit alors pas un groupe nitro.

2. Composé suivant la revendication 1, dans lequel:

A est le groupe nitro;

D est choisi entre l'hydrogène, le groupe nitro, cyano, un groupe alkyle en $C_1$ à $C_6$; alkoxy en $C_1$ à $C_6$ et halogénalkoxy en $C_1$ à $C_6$;

$R^1$ est choisi entre l'hydrogène, un groupe alkyle en $C_1$ à $C_6$, alcényle en $C_2$ à $C_6$, alcynyle en $C_2$ à $C_6$ et le groupe benzyle;

$R^2$ est choisi entre l'hydrogène et un groupe alkyle en $C_1$ à $C_6$;

B, E, T, U, V et $R^3$ représentent l'hydrogène;

W est choisi entre les groupes

$$-\overset{\overset{\displaystyle O}{\|}}{C}-G \quad \text{et} \quad -CH_2Z$$

dans lesquels: G est choisi entre les radicaux hydroxy, mercapto, alkoxy en $C_1$ à $C_{10}$, alcényloxy en $C_2$ à $C_{10}$, alcynyloxy en $C_2$ à $C_{10}$, cycloalkoxy en $C_3$ à $C_7$, alkylthio en $C_1$ à $C_{10}$, benzyloxy, N,N-di-(alkyle en $C_1$ à $C_6$)-amino, le groupe OM dans lequel M est un ion de métal alcalin ou de métal alcalino-terreux et un groupe alkoxy en $C_1$ à $C_{10}$ portant un substituant choisi entre des radicaux alkoxy en $C_1$ à $C_6$, alkylthio en $C_1$ à $C_6$, halogèno, N,N-di-(alkyle en $C_1$ à $C_6$)-amino, N,N,N-tri-(alkyle en $C_1$ à $C_6$)-ammonio et morpholino; et Z est choisi entre le groupe hydroxy et un groupe alkoxy en $C_1$ à $C_6$;

X est l'oxygène; et

*n* est égal à 0.

3. Composé suivant la revendication 1, dans lequel:

A est choisi entre l'hydrogène, un halogène, les groupes halogénalkyle en $C_1$ à $C_6$, cyano et amino;

D est choisi entre l'hydrogène, le groupe nitro, cyano, un halogène, les groupes alkyle en $C_1$ à $C_6$, halogénalkyle en $C_1$ à $C_6$, alkoxy en $C_1$ à $C_6$ et halogénalkoxy en $C_1$ à $C_6$;

$R^1$ est choisi entre l'hydrogène et les groupes alkyle en $C_1$ à $C_6$, alcényle en $C_2$ à $C_6$, alcynyle en $C_2$ à $C_6$ et benzyle;

$R^2$ est choisi entre l'hydrogène et un groupe alkyle en $C_1$ à $C_6$,

B, E, T, U, V et $R^3$ sont de l'hydrogène;

W est choisi entre les groupes

$$-\overset{\overset{\displaystyle O}{\|}}{C}-G \quad \text{et} \quad -CH_2Z$$

dans lesquels: G est choisi entre un groupe hydroxy, mercapto, alkoxy en $C_1$ à $C_{10}$, alcényloxy en $C_2$ à $C_{10}$, alcynyloxy en $C_2$ à $C_{10}$, cyclohexyloxy, alkylthio en $C_2$ à $C_{10}$, benzyloxy, N,N-di-(alkyle en $C_1$ à $C_6$)-amino, le groupe OM dans lequel M est un ion de métal alcalin ou alcalino-terreux et un groupe alkoxy en $C_1$ à $C_{10}$ substitué avec un substituant choisi entre des radicaux alkoxy en $C_1$ à $C_6$, alkylthio en $C_1$ à $C_6$, halogéno, N,N-di-(alkyle en $C_1$ à $C_6$)-amino, N,N,N-tri-(alkyle en $C_1$ à $C_6$)-ammonio et morpholino; et Z est choisi entre le groupe hydroxy et un groupe alkoxy en $C_1$ à $C_6$;

X est l'oxygène; et

*n* est égal à 0.

4. Composé suivant la revendication 1, dans lequel:

A est le groupe nitro,

D est choisi entre un groupe halogénalkyle en $C_1$ à $C_6$ et un halogène;

$R^1$ est choisi entre l'hydrogène et des groupes alkyle en $C_1$ à $C_6$, alcényle en $C_2$ à $C_6$ et le groupe benzyle;

$R^2$ est choisi entre l'hydrogène et un groupe alkyle en $C_1$ à $C_6$;

B, E, T, U, V et $R^3$ sont de l'hydrogène;

W est choisi entre les groupes

$$-\overset{\displaystyle O}{\overset{\|}{C}}-G \quad \text{et} \quad -CH_2Z$$

dans lesquels G est choisi entre des radicaux alcényloxy en $C_2$ à $C_{10}$, alcynyloxy en $C_2$ à $C_{10}$, cycloalkoxy en $C_3$ à $C_7$, benzyloxy, N,N-di-(alkyle en $C_1$ à $C_6$)-amino, alkoxy en $C_1$ à $C_{10}$ substitué avec un substituant choisi entre des radicaux alkoxy en $C_1$ à $C_6$, alkylthio en $C_1$ à $C_6$, halogéno, N,N-di-(alkyle en $C_1$ à $C_6$)-amino, N,N,N-tri-(alkyle en $C_1$ à $C_6$)ammonio et morpholino; et Z est choisi entre un groupe hydroxy et alkoxy en $C_1$ à $C_6$;

X est l'oxygène; et

*n* est égal à 0.

5. Composé suivant la revendication 1, dans lequel:

A est le groupe nitro;

D est choisi entre des groupes halogénalkyle en $C_1$ à $C_6$ et un halogène;

$R^1$ est un groupe alcynyle en $C_2$ à $C_6$;

$R^2$ est choisi entre l'hydrogène et un groupe alkyle en $C_1$ à $C_6$;

B, E, T, U, V et $R^3$ représentent l'hydrogène;

W est choisi entre les groupes

$$-\overset{\displaystyle O}{\overset{\|}{C}}-G \quad \text{et} \quad -CH_2Z$$

dans lesquels: G est choisi entre les groupes hydroxy, mercapto, alkoxy en $C_1$ à $C_6$, alcényloxy en $C_2$ à $C_6$, alcynyloxy en $C_2$ à $C_{10}$, cycloalkoxy en $C_3$ à $C_7$, alkylthio en $C_1$ à $C_{10}$, benzyloxy, N,N-di-(alkyle en $C_1$ à $C_6$)-amino, le groupe OM dans lequel M est un ion de métal alcalin ou de métal alcalino-terreux et le groupe alkoxy en $C_1$ à $C_6$ portant un substituant choisi entre les radicaux alkoxy en $C_1$ à $C_6$, alkylthio en $C_1$ à $C_6$, halogéno, N,N-di-(alkyle ne $C_1$ à $C_6$)-amino, N,N,N-tri-(alkyle en $C_1$ à $C_6$)-ammonio et morpholino; et Z est choisi entre le groupe hydroxy et un groupe alkoxy en $C_1$ à $C_6$;

X est l'oxygène; et

*n* est égal à 0.

6. Composé suivant la revendication 1 ou 2, dans lequel:

A est le groupe nitro;

D est choisi entre les groupes nitro, cyano, méthyle, éthyle, méthoxy et éthoxy;

$R^1$ est choisi entre l'hydrogène et les groupes méthyle, éthyle, n-propyle, n-butyle, allyle, propargyle et benzyle;

$R^2$ est le groupe méthyle;

B, E, T, U, V et $R^3$ sont de l'hydrogène;

W est choisi entre les groupes

$$-\overset{\displaystyle O}{\overset{\|}{C}}-G \quad \text{et} \quad -CH_2Z$$

dans lesquels: G est choisi entre les groupes hydroxy, alkoxy en $C_1$ à $C_6$, allyloxy, propargyloxy, alkylthio en $C_1$ à $C_4$, cyclohexyloxy, benzyloxy, N,N-diméthylamino, N,N-diéthylamino, 2-halogénéthoxy, 2-(alkylthio en $C_1$ à $C_6$)-éthoxy, 2-(morpholino)-éthoxy, 2-[N,N-di-(alkyle en $C_1$ à $C_6$)-amino]-éthoxy, 2-[N,N,N-tri-(alkyle en $C_1$ à $C_6$)-ammonio]-éthoxy et les groupes OM dans lesquels M est le sodium ou le potassium; et Z est choisi entre le groupe hydroxy et des groupes alkoxy en $C_1$ à $C_6$;

X est l'oxygène; et

*n* est égal à 0.

7. Composé suivant la revendication 1 ou 3, dans lequel:

A est choisi entre l'hydrogène et des radicaux fluoro, chloro, bromo, trifluorométhyle et amino;

D est choisi entre les radicaux fluoro, chloro, bromo, nitro et trifluorométhyle;

$R^1$ est choisi entre l'hydrogène et les radicaux méthyle, éthyle, n-propyle, n-butyle, allyle, propargyle et benzyle;

$R^2$ est le radical méthyle;

B, E, T, U, V et $R^3$ sont de l'hydrogène;

W est choisi entre les groupes

$$-\overset{\displaystyle O}{\overset{\|}{C}}-G \quad \text{et} \quad -CH_2Z$$

dans lesquels: G est choisi dans le groupe comprenant les radicaux hydroxy, alkoxy en $C_1$ à $C_6$, allyloxy, propargyloxy, cyclohexyloxy, benzyloxy, N,N-diméthylamino, N,N-diéthylamino, 2-halogénéthoxy, 2-alkylthio en $C_1$ à $C_6$)-éthoxy, 2-(morpholino)-éthoxy, 2-[N,N-di-(alkyle en $C_1$ à $C_6$)-amino]-éthoxy, 2-[N,N,N-tri-(alkyle en $C_1$ à $C_6$)-ammonio]-éthoxy et le groupe OM dans lequel M est le sodium ou le potassium; et Z est choisi entre le groupe hydroxy et des groupes alkoxy en $C_1$ à $C_6$;

X est l'oxygène; et

*n* est égal à 0.

8. Composé suivant la revendication 1 ou 4, dans lequel:

A est le groupe nitro;

D est choisi entre les radicaux fluoro, chloro, bromo et trifluorométhyle;

$R^1$ est choisi entre l'hydrogène et les radicaux méthyle, éthyle, n-propyle, n-butyle, allyle et benzyle;

$R^2$ est le radical méthyle;

B, E, T, U, V et $R^3$ sont de l'hydrogène;

W est choisi entre les groupes

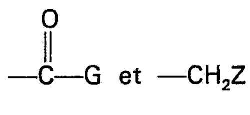

dans lesquels: G est choisi entre les radicaux allyloxy, propargyloxy, cyclohexyloxy, benzyloxy, N,N-diméthylamino, N,N-diéthylamino, 2-halogénéthoxy, 2-alkylthio en $C_1$ à $C_6$)-éthoxy, 2-(morpholino)-éthoxy, 2-[N,N-di-(alkyle en $C_1$ à $C_6$)-amino]-éthoxy et 2-[N,N,N-tri-(alkyle en $C_1$ à $C_6$)-ammonio]-éthoxy; et Z est choisi entre les radicaux hydroxy et alkoxy en $C_1$ à $C_6$;

X est l'oxygène; et

*n* est égal à 0.

9. Composé suivant la revendication 1 ou 5, dans lequel:

A est le groupe nitro;

D est choisi entre les radicaux fluoro, chloro, bromo et trifluorométhyle;

$R^1$ est le groupe propargyle;

$R^2$ est le groupe méthyle;

B, E, T, U, V et $R^3$ sont de l'hydrogène;

W est choisi entre les groupes

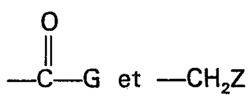

dans lesquels: G est choisi entre les groupes hydroxy, alkoxy en $C_1$ à $C_6$, allyloxy, propargyloxy, cyclohexyloxy, benzyloxy, N,N-diméthylamino, N,N-diéthylamino, 2-halogénéthoxy, 2-(alkylthio en $C_1$ à $C_6$)-éthoxy, 2-[N,N-di-(alkyle en $C_1$ à $C_6$)-amino]-éthoxy et 2-[N,N,N-tri-(alkyle en $C_1$ à $C_6$)-ammonio]-$C_6$)-ammonio]-éthoxy et le groupe OM dans lequel M est le sodium ou le potassium; et Z est choisi entre les groupes hydroxy et alkoxy en $C_1$ à $C_6$;

X est l'oxygène; et

*n* est égal à 0.

10. Composé suivant l'une quelconque des revendications 1, 2 et 6, choisi dans le groupe comprenant:

le 2-[4-(2,4-dinitro-anilino)-phénoxy]-propionate de méthyle;

le 2-[4-(4-méthoxy-2-nitro-anilino)-phénoxy]-propionate de méthyle;

le 2-[4-(4-cyano-2-nitro-anilino)phénoxy]-propionate de méthyle; et

le 2-[4-(4-cyano-N-méthyl-2-nitro-anilino)-phénoxy]-propionate d'éthyle.

11. Composé suivant l'une quelconque des revendications 1, 3 et 7, choisi dans le groupe comprenant:

le 2-[4-(2,4-dichloro-N-méthylanilino)-phénoxy]-propionate de méthyle;

le 2-[4-(2,4-dichloro-anilino)-phénoxy]-propionate de méthyle; et

le 2-[4-(4-nitro-anilino)-phénoxy]-propionate de méthyle.

12. Composé suivant l'une quelconque des revendications 1, 4 et 8, dans lequel:

A est le groupe nitro;

D est choisi entre le radical chloro et le groupe trifluorométhyle;

$R^1$ est choisi entre l'hydrogène et le groupe méthyle;

$R^2$ est le groupe méthyle;

B, E, T, U, V et $R^3$ sont de l'hydrogène;

W est choisi entre les groupes

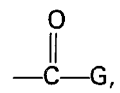

où G est choisi entre les radicaux allyloxy, propargyloxy, cyclohexyloxy, 2-(N,N-diméthylamino)-éthoxy, 2-(N,N-diéthylamino)-éthoxy, iodure de 2-(N,N,N-triméthylammonio)éthoxy et 2-brométhoxy; et les groupes $-CH_2Z$ dans lesquels Z est le radical éthoxy;

X est l'oxygène; et

*n* est égal à 0.

13. Composé suivant l'une quelconque des revendications 1, 5 et 9, qui est le 2-[4-(2-nitro-N-propargyl-4-trifluorométhylanilino)-phénoxy]-propionate de méthyle.

14. Composition herbicide, comprenant comme ingrédient actif un composé tel que défini conformément à l'une quelconque des revendications 1 à 13 inclus et un support approprié.

15. Composition suivant la revendication 14, sous la forme d'un liquide et comprenant un agent tensioactif.

16. Composition suivant la revendication 14, sous la forme d'une poudre.

17. Composition diluée suivant l'une quelconque des revendications 14 à 16, qui comprend 0,01 à 2% en poids d'ingrédient actif.

18. Composition concentrée suivant l'une quelconque des revendications 14 à 16, qui comprend 20 à 90% en poids d'ingrédient actif.

19. Procédé pour altérer gravement ou détruire des plantes indésirables, qui consiste à appliquer auxdites plantes ou au milieu de croissance desdites plantes, une quantité efficace d'un composé tel que défini suivant l'une quelconque des revendications 1 à 13 ou une quantité efficace d'une composition telle que définie conformément à l'une quelconque des revendications 14 à 18 inclus.

20. Procédé pour combattre sélectivement la croissance de mauvaises herbes monocotylédones dans des cultures de dicotylédones, qui consiste à appliquer auxdites plantes cultivées ou à leur milieu de croissance un composé tel que défini suivant l'une quelconque des revendications 1 à 13 inclus ou une composition telle que définie suivant l'une quelconque des revendications 14 à 18 inclus, en une quantité suffisante pour altérer gravement ou détruire les mauvaises herbes, mais insuffisante pour atteindre sensiblement la plante cultivée.

21. Procédé suivant la revendication 19 ou 20, dans lequel le composé est appliqué à un taux compris dans la plage de 0,005 à 20 kg/ha.

22. Procédé suivant la revendication 21, dans lequel le taux d'application se situe dans la plage de 0,05 à 5 kg/ha.

23. Procédé de synthèse d'un composé de formule I suivant l'une quelconque des revendications 1 à 13 inclus, qui comprend la condensation, en présence d'une matière alcaline, d'un phénol ou d'un thiophénol de formule IX avec un composé de formule X, dans laquelle hal est le chlore, le brome ou l'iode.

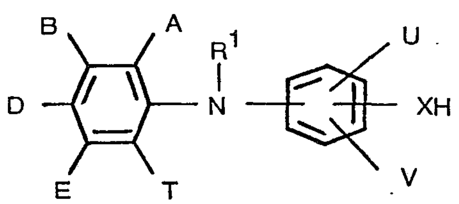

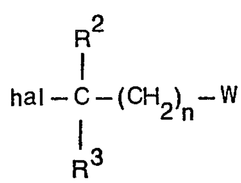

IX

24. Procédé de synthèse d'un composé de formule I tel que défini suivant l'une quelconque des revendications 1 à 13 inclus, qui comprend la condensation d'un composé de formule V, dans laquelle L est un groupe partant, avec une aniline de formule VI.

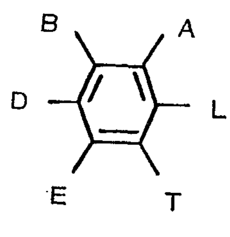

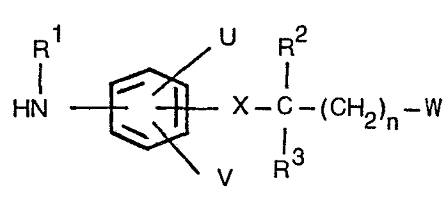

V VI

25. Procédé de synthèse d'un composé de formule I tel que défini suivant l'une quelconque des revendications 1 à 13 inclus, qui comprend les étapes successives ci-après:

a) condensation d'un composé de formule V, dans laquelle L est un groupe partant, avec une aniline de formule VII dans laquelle Q est un groupe hydroxy, mercapto, alkoxy en $C_1$ à $C_6$ ou alkylthio en $C_1$ à $C_6$ pour former un composé de formule VIII;

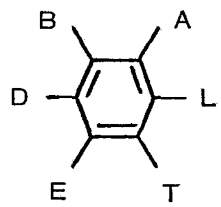

V

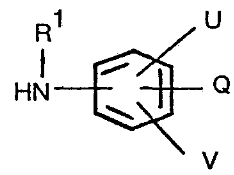

VII

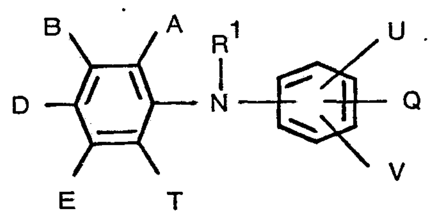

VIII

b) désalkylation du composé de formule VIII, dans laquelle Q est un groupe alkoxy en $C_1$ à $C_6$ ou alkylthio en $C_1$ à $C_6$ pour former un composé de formule IX; et

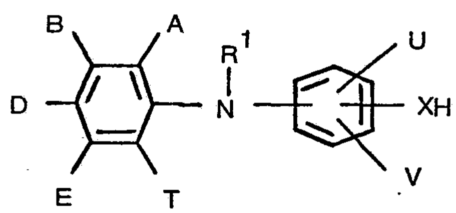

IX

c) condensation, en présence d'une matière alcaline, d'un composé de formule IX obtenu dans l'étape a) ou dans l'étape b) ci-dessus avec un composé de formule X, dans laquelle hal est le chlore, le brome ou l'iode.

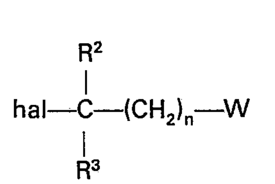

X

**Patentansprüche**

1. Verbindung der Formel II

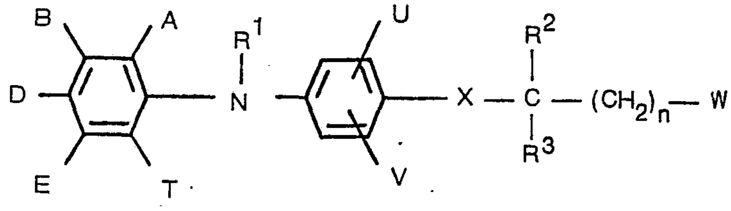

II

oder ein Salz davon, worin

A, B, D, E und T unabhängig ausgewählt sind aus Wasserstoff, Halogen, Nitro, Cyano, Amino, Formyl, $C_1$—$C_6$-Alkyl, $C_1$—$C_6$-Alkoxy, $C_1$—$C_6$-Halogenalkyl, $C_1$—$C_6$-Halogenalkoxy, ($C_1$—$C_6$-Alkoxy)-carbonyl, $C_1$—$C_6$-Halogenalkylthio, $C_1$—$C_6$-Halogenalkylsulfinyl, $C_1$—$C_6$-Alkylsulfonyl, $C_1$—$C_6$-Halogenalkylsulfonyl, Sulfo, $C_1$—$C_6$-Alkoxysulfonyl, N,N-Di($C_1$—$C_6$-alkyl)carbamoyl, Sulfamoyl und N,N-Di($C_1$—$C_6$-alkyl)sulfamoyl,

U und V unabhängig ausgewählt sind aus Wasserstoff, Halogen und Nitro,

$R^1$ ausgewählt ist aus Wasserstoff, $C_1$—$C_6$-Alkyl, $C_2$—$C_6$-Alkenyl, $C_2$—$C_6$-Alkinyl, $C_2$—$C_6$-Alkanoyl, Benzyl, Benzoyl und Halogenbenzoyl,

$R^2$ und $R^3$ unabhängig ausgewählt sind aus Wasserstoff und $C_1$—$C_6$-Alkyl,

W ausgewählt ist aus den Gruppen

$$-\overset{O}{\overset{\|}{C}}-G \text{ und } -CH_2Z,$$

wobei G ausgewählt ist aus Hydroxy, Mercapto, $C_1$—$C_{10}$-Alkoxy, $C_2$—$C_{10}$-Alkenyloxy, $C_2$—$C_{10}$-Alkinyloxy, $C_3$—$C_7$-Cycloalkoxy, $C_1$—$C_{10}$-Alkylthio, Benzyloxy, N,N-Di($C_1$—$C_6$-alkyl)amino, $C_1$—$C_{10}$-Alkoxy, das mit einem der Substituenten Halogen, Amino, Ammonio, N-($C_1$—$C_6$-Alkyl)amino, N,N-Di($C_1$—$C_6$-Alkyl)amino, N,N,N-Tri($C_1$—$C_6$-alkyl)ammonio, $C_1$—$C_6$-Alkoxy, $C_1$—$C_6$-Alkylthio, Morpholino und 2-Pyridyl substituiert ist, und der Gruppe OM, wobei M ein Alkalimetall- oder Erdalkalimetallion ist, und Z ausgewählt ist aus Hydroxy und $C_1$—$C_6$-Alkoxy,

X für Sauerstoff steht und

n für 0 oder 2 steht;

mit der Maßgabe, daß, wenn

B, E, T, U, V und $R^3$ für Wasserstoff stehen,

D für Trifluoromethyl oder Halogen steht,

$R^1$ für Wasserstoff, $C_1$—$C_6$-Alkyl, $C_2$—$C_6$-Alkenyl oder Benzyl steht,

$R^2$ für $C_1$—$C_6$-Alkyl steht,

W für die Gruppe

$$-\overset{O}{\overset{\|}{C}}-G$$

steht, wobei G ausgewählt ist aus Hydroxy, Mercapto, $C_1$—$C_{10}$-Alkoxy, $C_1$—$C_{10}$-Alkylthio oder der Gruppe OM, wobei M ein Alkalimetallion ist,

X für Sauerstoff steht und

N für 0 oder 2 steht,

A nicht Nitro bedeutet.

2. Verbindung nach Anspruch 1, worin

A für Nitro steht,

D ausgewählt ist aus Wasserstoff, Nitro, Cyano, $C_1$—$C_6$-Alkyl, $C_1$—$C_6$-Alkoxy und $C_1$—$C_6$-Halogenalkoxy,

$R^1$ ausgewählt ist aus Wasserstoff, $C_1$—$C_6$-Alkyl, $C_2$—$C_6$-Alkenyl, $C_2$—$C_6$-Alkinyl und Benzyl,

$R^2$ ausgewählt ist aus Wasserstoff und $C_1$—$C_6$-Alkyl,

B, E, T, U, V und $R^3$ für Wasserstoff stehen,

W ausgewählt ist aus den Gruppen

$$-\overset{O}{\overset{\|}{C}}-G \text{ und } -CH_2Z,$$

wobei G ausgewählt ist aus Wasserstoff, Mercapto, $C_1$—$C_{10}$-Alkoxy, $C_2$—$C_{10}$-Alkenyloxy, $C_2$—$C_{10}$-Alkinyloxy, $C_3$—$C_7$-Cycloalkoxy, $C_1$—$C_{10}$-Alkylthio, Benzyloxy, N,N-Di($C_1$—$C_6$-alkyl)amino, der Gruppe OM, wobei M ein Alkalimetall- oder Erdalkalimetallion ist, und $C_1$—$C_{10}$-Alkoxy, welches mit einem der Substituenten $C_1$—$C_6$-Alkoxy, $C_1$—$C_6$-Alkylthio, Halogen, N,N-Di($C_1$—$C_6$-alkyl)amino, N,N,N-Tri($C_1$—$C_6$-alkyl)ammonio und Morpholino substituiert ist, und Z ausgewählt ist aus Hydroxy und $C_1$—$C_6$-Alkoxy,

X für Sauerstoff steht und

n für 0 steht.

3. Verbindung nach Anspruch 1, worin

A ausgewählt ist aus Wasserstoff, Halogen, $C_1$—$C_6$-Halogenalkyl, Cyano und Amino,

D ausgewählt ist aus Wasserstoff, Nitro, Cyano, Halogen, $C_1$—$C_6$-Alkyl, $C_1$—$C_6$-Halogenalkyl, $C_1$—$C_6$-Alkoxy und $C_1$—$C_6$-Halogenalkoxy,

$R^1$ ausgewählt ist aus Wasserstoff, $C_1$—$C_6$-Alkyl, $C_2$—$C_6$-Alkenyl, $C_2$—$C_6$-Alkinyl und Benzyl,
$R^2$ ausgewählt ist aus Wasserstoff und $C_1$—$C_6$-Alkyl,
B, E, T, U, V und $R^3$ für Wasserstoff stehen,
W ausgewählt ist aus den Gruppen

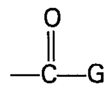 und —$CH_2Z$,

wobei G ausgewählt ist aus Hydroxy, Mercapto, $C_1$—$C_{10}$-Alkoxy, $C_2$—$C_{10}$-Alkenyloxy, $C_2$—$C_{10}$-Alkinyloxy, Cyclohexyloxy, $C_2$—$C_{10}$-Alkylthio, Benzyloxy, N,N-Di($C_1$—$C_6$-alkyl)amino, der Gruppe OM, wobei M ein Alkalimetall- oder Erdalkalimetallion ist, und $C_1$—$C_{10}$-Alkoxy, das mit einem der Substituenten $C_1$—$C_6$-Alkoxy, $C_1$—$C_6$-Alkylthio, Halogen, N,N-Di($C_1$—$C_6$-alkyl)amino, N,N,N-Tri($C_1$—$C_6$-alkyl)ammonio und Morpholino substituiert ist, und Z ausgewählt ist aus Hydroxy und $C_1$—$C_6$-Alkoxy,
X für Sauerstoff steht und
N für 0 steht.

4. Verbindung nach Anspruch 1, worin
A für Nitro steht,
D ausgewählt ist aus $C_1$—$C_6$-Halogenalkyl und Halogen,
$R^1$ ausgewählt ist aus Wasserstoff, $C_1$—$C_6$-Alkyl, $C_2$—$C_6$-Alkenyl und Benzyl,
$R^2$ ausgewählt ist aus Wasserstoff und $C_1$—$C_6$-Alkyl,
B, E, T, U, V und $R^3$ für Wasserstoff stehen,
W ausgewählt ist aus den Gruppen

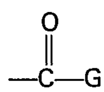 und —$CH_2Z$,

wobei G ausgewählt ist aus $C_2$—$C_{10}$-Alkenyloxy, $C_2$—$C_{10}$-Alkinyloxy, $C_3$—$C_7$-Cycloalkoxy, Benzyloxy, N,N-Di($C_1$—$C_6$-alkyl)amino und $C_1$—$C_{10}$-Alkoxy, das mit einem der Substituenten $C_1$—$C_6$-Alkoxy, $C_1$—$C_6$-Alkylthio, Halogen, N,N-Di($C_1$—$C_6$-alkyl)amino, N,N,N-Tri($C_1$—$C_6$-alkyl)ammonio und Morpholino substituiert ist, und Z ausgewählt ist aus Hydroxy und $C_1$—$C_6$-Alkoxy,
X für Sauerstoff steht und
n für 0 steht.

5. Verbindung nach Anspruch 1, worin
A für Nitro steht,
D ausgewählt ist aus $C_1$—$C_6$-Halogenalkyl und Halogen,
$R^1$ für $C_2$—$C_6$-Alkinyl steht,
$R^2$ ausgewählt ist aus Wasserstoff und $C_1$—$C_6$-Alkyl,
B, E, T, U, V und $R^3$ für Wasserstoff stehen,
W ausgewählt ist aus den gruppen

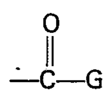 und —$CH_2Z$,

wobei G ausgewählt ist aus Hydroxy, Mercapto, $C_1$—$C_6$-Alkoxy, $C_2$—$C_{10}$-Alkenyloxy, $C_2$—$C_{10}$-Alkinyloxy, $C_3$—$C_7$-Cycloalkoxy, $C_1$—$C_{10}$-Alkylthio, Benzyloxy, N,N-Di($C_1$—$C_6$-alkyl)amino, der Gruppe OM, wobei M ein Alkalimetall- oder Erdalkalimetallion ist, und $C_1$—$C_6$-Alkoxy, das mit einem der Substituenten $C_1$—$C_6$-Alkoxy, $C_1$—$C_6$-Alkylthio, Halogen, N,N-Di($C_1$—$C_6$-alkyl)amino, N,N,N-Tri($C_1$—$C_6$-alkyl)ammonio und Morpholino substituiert ist, und Z ausgewählt ist aus Hydroxy und $C_1$—$C_6$-Alkoxy,
X für Sauerstoff steht und
n für 0 steht.

6. Verbindung nach Anspruch 1 oder 2, worin
A für Nitro steht,
D ausgewählt ist aus Nitro, Cyano, Methyl, Äthyl, Methoxy und Äthoxy,
$R^1$ ausgewählt ist aus Wasserstoff, Methyl, Äthyl, n-Propyl, n-Butyl, Allyl, Propargyl und Benzyl,
$R^2$ für Methyl steht,
B, E, T, U, V und $R^3$ für Wasserstoff stehen,
W ausgewählt ist aus den Gruppen

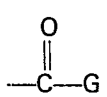 und —$CH_2Z$,

wobei G ausgewählt ist aus Hydroxy, $C_1$—$C_6$-Alkoxy, Allyloxy, Propargyloxy, $C_1$—$C_4$-Alkylthio, hexyloxy, Benzyloxy, N,N-Dimethylamino, N,N-Diäthylamino, 2-Halogenäthoxy, 2-($C_1$—$C_6$-Alkylthio)-äthoxy, 2-(Morpholino)äthoxy, 2-[N,N-Di($C_1$—$C_6$-Alkyl)amino]äthoxy, 2-[N,N,N-Tri($C_1$—$C_6$-alkyl)-ammonio]äthoxy und der Gruppe OM, wobei M Natrium oder Kalium ist, und Z ausgewählt ist aus Hydroxy und $C_1$—$C_6$-Alkoxy,

X für Sauerstoff steht und

n für 0 steht.

7. Verbindung nach Anspruch 1 oder 3, worin

A ausgewählt ist aus Wasserstoff, Fluoro, Chloro, Bromo, Trifluoromethyl und Amino,

D ausgewählt ist aus Fluoro, Chloro, Bromo, Nitro und Trifluoromethyl,

$R^1$ ausgewählt ist aus Wasserstoff, Methyl, Äthyl, n-Propyl, n-Butyl, Allyl, Propargyl und Benzyl,

$R^2$ für Methyl steht,

B, E, T, U, V und $R^3$ für Wasserstoff stehen,

W ausgewählt ist aus den Gruppen

$$-\overset{\overset{\displaystyle O}{\|}}{C}-G \quad \text{und} \quad -CH_2Z,$$

wobei G ausgewählt ist aus Hydroxy, $C_1$—$C_6$-Alkoxy, Allyloxy, Propargyloxy, Cyclohexyloxy, Benzyloxy, N,N-Dimethylamino, N,N-Diäthylamino, 2-Halogenäthoxy, 2-($C_1$—$C_6$-Alkylthio)äthoxy, 2-(Morpholino)-äthoxy, 2-[N,N-Di($C_1$—$C_6$-alkyl)amino]äthoxy, 2-[N,N,N-Tri($C_1$—$C_6$-alkyl)ammonio]äthoxy und der Gruppe OM, wobei M Natrium oder Kalium ist, und Z ausgewählt ist aus Hydroxy und $C_1$—$C_6$-Alkoxy,

X für Sauerstoff steht und

n für 0 steht.

8. Verbindung nach Anspruch 1 oder 4, worin

A für Nitro steht,

D ausgewählt ist aus Fluoro, Chloro, Bromo und Trifluoromethyl,

$R^1$ ausgewählt ist aus Wasserstoff, Methyl, Äthyl, n-Propyl, n-Butyl, Allyl und Benzyl,

$R^2$ für Methyl steht,

B, E, T, U, V und $R^3$ für Wasserstoff stehen,

W ausgewählt ist aus den Gruppen

$$-\overset{\overset{\displaystyle O}{\|}}{C}-G \quad \text{und} \quad -CH_2Z,$$

wobei G augewählt ist aus Allyloxy, Propargyloxy, Cyclohexyloxy, Benzyloxy, N,N-Dimethylamino, N,N-Diäthylamino, 2-Halogenäthoxy, 2-($C_1$—$C_6$-Alkylthio)äthoxy, 2-(Morpholino)äthoxy, 2-[N,N-Di($C_1$—$C_6$-alkyl)amino]äthoxy und 2-[N,N,N-Tri($C_1$—$C_6$-alkyl)ammonio]äthoxy, und Z ausgewählt ist aus Hydroxy und $C_1$—$C_6$-Alkoxy,

X für Sauerstoff steht und

n für 0 steht.

9. Verbindung nach Anspruch 1 oder 5, worin

A für Nitro steht,

D ausgewählt ist aus Fluoro, Chloro, Bromo und Trifluoromethyl,

$R^1$ für Propargyl steht,

$R^2$ für Methyl steht,

B, E, T, U, V und $R^3$ für Wasserstoff stehen,

W ausgewählt ist aus den Gruppen

$$-\overset{\overset{\displaystyle O}{\|}}{C}-G \quad \text{und} \quad -CH_2Z,$$

wobei G ausgewält ist aus Hydroxy, $C_1$—$C_6$-Alkoxy, Allyloxy, Propargyloxy, Cyclohexyloxy, Benzyloxy, N,N-Dimethylamino, N,N-Diäthylamino, 2-Halogenäthoxy, 2-($C_1$—$C_6$-Alkylthio)äthoxy, 2-(Morpholino)-äthoxy, 2-[N,N-Di($C_1$—$C_6$-alkyl)amino]äthoxy, 2-[N,N,N-Tri($C_1$—$C_6$-alkyl)ammonio]äthoxy und der Gruppe OM, wobei M Natrium oder Kalium ist, und Z ausgewählt ist aus Hydroxy und $C_1$—$C_6$-Alkoxy,

X für Sauerstoff steht und

n für 0 steht.

10. Verbindung nach einem der Ansprüche 1, 2 und 6, welche ausgewählt ist aus:

2-[4-(2,4-Dinitroanilino)phenoxy]propionsäure-methylester;

2-[4-(4-Methoxy-2-nitroanilino)phenoxy]propionsäure-methylester;

2-[4-(4-Cyano-2-nitroanilino)phenoxy]propionsäure-methylester; und

2-[4-(4-Cyano-N-methyl-2-nitroanilino)phenoxy]propionsäure-äthylester.

11. Verbindung nach einem der Ansprüche 1, 3 und 7, welche ausgewählt ist aus:
2-[4-(2,4-Dichloro-N-methylanilino)phenoxy]propionsäure-methylester;
2-[4-(2,4-Dichloroanilino)phenoxy]propionsäure-methylester; und
2-[4-(4-Nitroanilinophenoxy]propionsäure-methylester.

12. Verbindung nach einem der Annsprüche 1, 4 und 8, worin:
A für Nitro steht,
D ausgewählt ist aus Chloro und Trifluoromethyl,
$R^1$ ausgewählt ist aus Wasserstoff und Methyl;
$R^2$ für Methyl steht,
B, E, T, U, V und $R^3$ für Wasserstoff stehen,
W ausgewählt ist aus den Gruppen

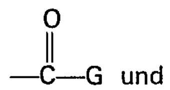 und $—CH_2Z$,

wobei G ausgewählt ist aus Allyloxy, Propargyloxy, Cyclohexyloxy, 2-(N,N-Dimethylamino)äthoxy, 2 (N,N-Diäthylamino)äthoxy, 2-(N,N,N-Trimethylammonio)äthoxyjodid und 2-Bromoäthoxy, und Z fü Äthoxy steht,
X für Sauerstoff steht und
n für 0 steht.

13. Verbindung nach einem der Ansprüche 1, 5 und 9, nämlich:
2-[4-(2-Nitro-N-propargyl-4-trifluoromethylanilino)phenoxy]propionsäure-methylester.

14. Herbicide Zusammensetzung, welche als aktiven Bestandteil eine Verbindung nach einem der Ansprüche 1 bis 13 und einen Träger hierfür enthält.

15. Zusammensetzung nach Anspruch 14, wobei die Zusammensetzung in Form einer Flüssigkeit vorliegt und ein oberflächenaktives Mittel enthält.

16. Zusammensetzung nach Anspruch 14, wobei die Zusammensetzung in Form eines Pulvers vorliegt.

17. Verdünnte Zusammensetzung nach einem der Ansprüche 14 bis 16, welche 0,01 bis 2 Gew.-% des aktiven Bestandteils enthält.

18. Konzentrierte Zusammensetzung nach einem der Ansprüche 14 bis 16, welche 20 bis 90 Gew.-% aktiven Bestandteil enthält.

19. Verfahren zum starken Schädigen oder Abtöten unerwünschter Pflanzen, bei welchem auf die Pflanzen oder auf das Wachstumsmedium der Pflanzen eine wirksame Menge einer Verbindung nach einem der Ansprüche 1 bis 13 oder eine wirksame Menge einer Zusammensetzung nach einem der Ansprüche 14 bis 18 aufgebracht wird.

20. Verfahren zur selektiven Beeinflussung des Wachstums von einkeimblättrigem Unkraut in zweikeimblätttrigen Anpflanzungen, bei welchem auf die Anpflanzung oder auf das Wachstumsmedium der Anpflanzung eine Verbindung nach einem der Ansprüche 1 bis 13 oder eine Zusammensetzung nach einem der Ansprüche 14 bis 18 in einer Menge aufgebracht wird, die ausreicht, das Unkraut stark zu schädigen oder abzutöten, die aber nicht ausreicht, die Anpflanzung wesentlich zu schädigen.

21. Verfahren nach Anspruch 19 oder 20, bei welchem die Verbindung in einer Rate im Bereich von 0,005 bis 20 kg/ha aufgebracht wird.

22. Verfahren nach Anspruch 21, bei welchem die Rate im Bereich von 0,05 bis 5 kg/ha liegt.

23. Verfahren zur Synthese einer in einem der Ansprüche 1 bis 13 definierten Verbindung der Formel II, bei welchem ein Phenol oder Thiophenol der Formel IX mit einer Verbindung der Formel X, worin hal für Chlor, Brom oder Jod steht, in Gegenwart eines alkalischen Materials kondensiert wird.

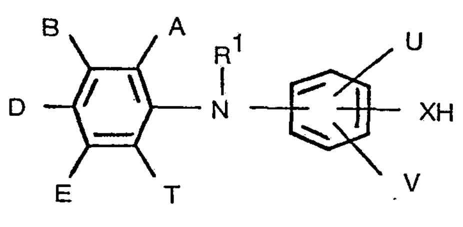

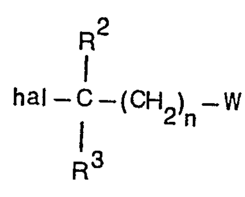

IX

24. Verfahren zur Synthese einer in einem der Ansprüche 1 bis 13 definierten Verbindung der Formel II, bei welchem eine Verbindung der Formel V, worin L für eine abspaltbare Gruppe steht, mit einem Anilin der Formel VI kondensiert wird.

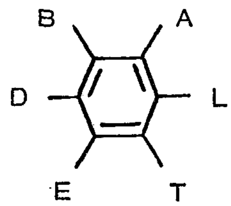

V

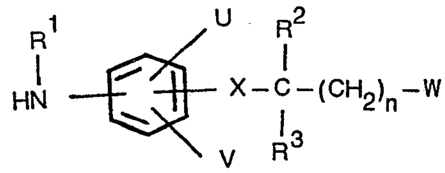

VI

25. Verfahren zur Synthese einer in einem der Ansprüche 1 bis 13 definierten Verbindung der Formel II, bei welchem nacheinander die folgenden Stufen ausgeführt werden:

a) Kondensation einer Verbindung der Formel V, worin L für eine abspaltbare Gruppe steht, mit einem Anilin der Formel VII, worin Q für Hydroxy, Mercapto, $C_1$—$C_6$-Alkoxy oder $C_1$—$C_6$-Alkylthio steht, wobei eine Verbindung dr Formel VIII entsteht;

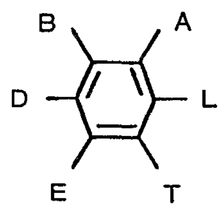

V

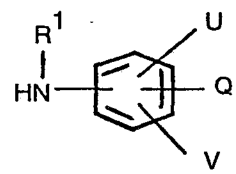

VII

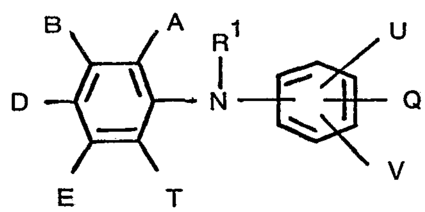

VIII

b) Dealkylierung der Verbindung der Formel VIII, worin Q für $C_1$—$C_6$-Alkoxy oder $C_1$—$C_6$-Alkylthio steht, wobei eine Verbindung der Formel IX entsteht; und

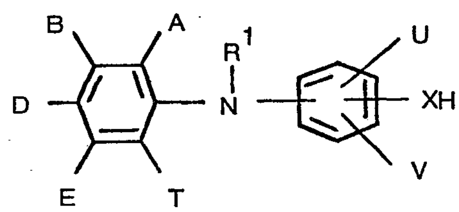

IX

c) Kondensation einer Verbindung der Formel IX, die in einer der obigen Stufen a) oder b) erhalten worden ist, mit einer Verbindung der Formel X, worin hal für Chlor, Brom oder Jod steht, in Gegenwart eines alkalischen Materials.

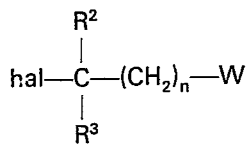

X